(19) Europäisches Patentamt
European Patent Office
Office européen des brevets

(11) Veröffentlichungsnummer: **0 264 019**
**A2**

(12) **EUROPÄISCHE PATENTANMELDUNG**

(21) Anmeldenummer: **87114212.1**

(22) Anmeldetag: **29.09.87**

(51) Int. Cl.4: **C07D 251/46** , C07D 251/22 , A01N 47/36 , //C07C154/02

(30) Priorität: **14.10.86 DE 3634927**

(43) Veröffentlichungstag der Anmeldung: **20.04.88 Patentblatt 88/16**

(84) Benannte Vertragsstaaten: **BE CH DE FR GB IT LI NL**

(71) Anmelder: **BAYER AG**

**D-5090 Leverkusen(DE)**

(72) Erfinder: **Fest, Christa, Dr.**
**Im Johannistal 20**
**D-5600 Wuppertal 1(DE)**

(54) **Verfahren zur Herstellung von Sulfonylisothioharnstoffen.**

(57) Man erhält die herbizid wirksamen Sulfonylisothioharnstoffe der allgemeinen Formel (I)

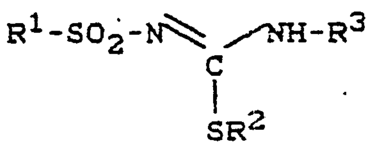 (I)

(worin die Reste $R^1$, $R^2$ und $R^3$ die in der Beschreibung angegebenen Bedeutungen haben) in guten Ausbeuten, indem man Iminodithiokohlensäureester der Formel (II),

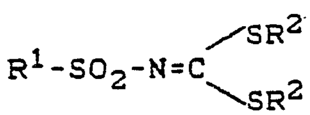 (II)

mit Aminen der Formel (III)

$R^3$-$NH_2$ (III)

in Gegenwart von Säureakzeptoren und gegebenenfalls in Gegenwart von Verdünnungsmitteln bei Temperaturen zwischen 0° C und 150° C umsetzt.

EP 0 264 019 A2

## Verfahren zur Herstellung von Sulfonylisothioharnstoffen

Die vorliegende Erfindun betrifft ein neues Verfahren zur Herstellung von Sulfonylisothioharnstoffen sowie neue Zwischenprodukte hierfür. Die Verfahrensprodukte sind bekannt und können als Herbizide verwendet werden.

Es ist bekannt, daß man Sulfonylisothioharnstoffe herstellen kann, indem man Sulfonylguanidine mit Mercaptan-Derivaten umsetzt. Nachteilig bei diesem Verfahren sind die unbefriedigenden Ausbeuten bei der Herstellung der einzusetzenden Sulfonylguanidine und/oder bei der Herstellung der Sulfonylisothioharnstoffe (vergl. z. B. EP-OS 173 957).

Es wurde nun gefunden, daß man Sulfonylisothioharnstoffe der allgemeinen Formel (I)

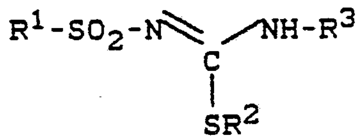

(I)

in welcher

$R^1$ für einen gegebenenfalls substituierten Rest aus der Reihe Aralkyl, Aryl und Heteroaryl steht,

$R^2$ für gegebenenfalls substituiertes Aryl steht und

$R^3$ für einen gegebenenfalls substituierten aromatischen oder heteroaromatischen Rest steht,

in guten Ausbeuten erhält, wenn man Iminodithiokohlensäureester der Formel (II)

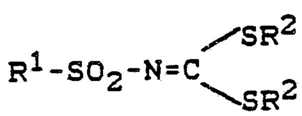

(II)

in welcher

$R^1$ und $R^2$ die oben angegebenen Bedeutungen haben,

mit Aminen der Formel (III)

$R^3\text{-}NH_2$ (III)

in welcher

$R^3$ die oben angegebenen Bedeutungen hat,

in Gegenwart von Säureakzeptoren und gegebenenfalls in Gegenwart von Verdünnungsmitteln bei Temperaturen zwischen 0 °C und 150 °C umsetzt.

Überraschenderweise gelingt es mit Hilfe des erfindungsgemäßen Verfahrens die Verbindungen der Formel (I) auf einfache Weise in guter Ausbeute herzustellen.

Bevorzugt werden mit den erfindungsgemäßen Verfahren die folgenden Verbindungen der Formel (I) hergestellt, in welcher

$R^1$ für den Rest

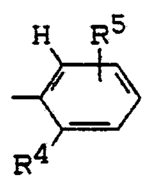

steht, worin

$R^4$ und $R^5$ gleich oder verschieden sind und für Wasserstoff, Halogen [wie insbesondere Fluor, Chlor, Brom und/oder Iod], Cyano, $C_1$-$C_6$-Alkyl, [welches gegebenenfalls durch Fluor, Chlor, Brom, Cyano, $C_1$-$C_4$-Alkoxy, $C_3$-$C_6$-Cycloalkyl oder Phenyl substituiert ist], für $C_1$-$C_4$-Alkoxy [welches gegebenenfalls durch Fluor, Chlor, Brom, Cyano oder $C_1$-$C_4$-Alkoxy substituiert ist], für Di-($C_1$-$C_4$-alkyl)-aminosulfonyl, für Phenyl oder Phenoxy, für $C_1$-$C_4$-Alkylcarbonylamino, $C_1$-$C_4$-Alkylamino-carbonyl-amino, Di-($C_1$-$C_4$-alkyl)-amino-carbonylamino, Di-($C_1$-$C_4$-alkyl)-aminocarbonyl oder für Di-($C_1$-$C_4$-alkyl)-aminosulfonylamino stehen, worin weiter

$R^1$ für den Rest

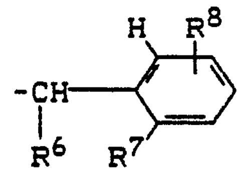

steht, worin

$R^6$ für Wasserstoff oder $C_1$-$C_4$-Alkyl steht,

$R^7$ und $R^8$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist] oder Di-($C_1$-$C_4$-alkyl)-aminosulfonyl stehen; worin weiter

$R^1$ für den Rest

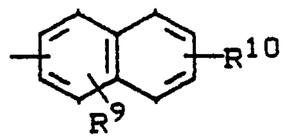

steht, worin

$R^9$ und $R^{10}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist] oder $C_1$-$C_4$-Alkoxy [welches gegebenenfalls durch Fluor und/oder Chlor substituiert ist], stehen; und

$R^2$ für einen Phenylrest steht, welcher gegebenenfalls substituiert ist durch einen oder mehrere Reste aus der Reihe

Halogen [wie insbesondere Fluor, Chlor, Brom und Iod], Cyano, Nitro, Hydroxy, Carboxy, $C_1$-$C_6$-Alkyl [welches gegebenenfalls durch Fluor, Chlor, Brom, Nitro, Cyano, Hydroxy, Carboxy, $C_1$-$C_4$-Alkoxy-carbonyl, $C_1$-$C_4$-Alkoxy, $C_1$-$C_4$-Alkylthio oder Phenyl substituiert ist], $C_3$-$C_6$-Cycloalkyl, $C_1$-$C_4$-Alkoxy [welches gegebenenfalls durch Fluor, Chlor, Brom, Cyano, Carboxy, $C_1$-$C_4$-Alkoxy, $C_1$-$C_4$-Alkylthio oder $C_1$-$C_4$-Alkoxy-carbonyl substituiert ist], $C_1$-$C_4$-Alkylthio [welches gegebenenfalls durch Fluor, Chlor, Brom, Cyano, Carboxy, $C_1$-$C_4$-Alkoxy-carbonyl substituiert ist], Amino, $C_1$-$C_4$-Alkyl-amino bzw. Di-($C_1$-$C_4$-alkyl)-amino [welches gegebenenfalls durch Fluor, Chlor, Brom, Cyano, Carboxy, $C_1$-$C_4$-Alkoxy oder $C_1$-$C_4$-Alkoxy-carbonyl substituiert sind], $C_1$-$C_4$-Alkyl-carbonyl-amino, $C_1C_4$-Alkoxy-carbonylamino, (Di)-$C_1$-$C_4$-Alkylamino-carbonyl-amino, Formyl, $C_1$-$C_4$-Alkyl-carbonyl, Benzoyl, $C_1$-$C_4$-Alkoxy-carbonyl, Phenoxy-carbonyl, Benzyloxycarbonyl, Phenyl [welches gegebenenfalls durch Fluor, Chlor, Brom, Cyano, Nitro, Hydroxy, oder Methyl substituiert ist], Phenoxy, Phenylthio, Phenylsulfonyl, Phenylamino oder Phenylazo [welches gegebenenfalls durch Fluor, Chlor, Brom, Cyano, Nitro, Methyl und/oder Trifluormethyl substituiert sind], Pyridoxy oder Pyrimidoxy [welches gegebenenfalls durch Fluor, Chlor, Brom, Cyano, Nitro, und/oder Trifluormethyl substituiert sind], $C_1$-$C_4$-Alkyl-carbonyloxy, $C_1$-$C_4$-Alkoxy-carbonyloxy, $C_1$-$C_4$-Alkyl-amino-carbonyloxy und Di-($C_1$-$C_4$-alkyl)-amino-carbonyloxy, oder welcher gegebenenfalls durch eine Alkylenkette [welche gegebenenfalls verzweigt und/oder durch ein oder mehrere Sauerstoffatome unterbrochen ist] oder einen Benzorest [welcher gegebenenfalls durch Fluor, Chlor, Brom, Cyano, Nitro, Methyl und/oder Trifluormethyl substituiert ist] anelliert ist; worin weiter

$R^3$ für den Rest

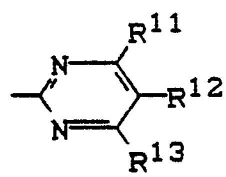

steht, worin

$R^{11}$ für Wasserstoff, Fluor, Chlor, Brom, Hydroxy, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio [welches gegebenenfalls durch Fluor und/oder Chlor substituiert ist], $C_1$-$C_4$-Alkylamino oder Di-($C_1$-$C_4$-alkyl)-amino steht,

$R^{12}$ für Wasserstoff, Fluor, Chlor, Brom, $C_1$-$C_4$-Alkyl [welches gegebenenfalls durch Fluor und/oder Chlor

substituiert ist], Cyano, Formyl, $C_1$-$C_4$-Alkyl-carbonyl oder $C_1$-$C_4$-Alkoxycarbonyl steht und
$R^{13}$ für Wasserstoff, Fluor, Chlor, Brom, Hydroxy, $C_1$-$C_4$-Alkyl [welches gegebenenfalls durch Fluor und oder Chlor substituiert ist], $C_1$-$C_4$-Alkoxy [welches gegebenenfalls durch Fluor und/oder Chlor substituiert ist], $C_1$-$C_4$-Alkylthio [welches gegebenenfalls durch Fluor und/oder Chlor substituiert ist], $C_1$-$C_4$-Alkyl-amino oder Di-($C_1$-$C_4$-alkyl)-amino steht, oder
$R^{12}$ und $R^{13}$ gemeinsam für $C_3$-$C_4$-Alkandiyl stehen; worin weiter
$R^3$ für den Rest

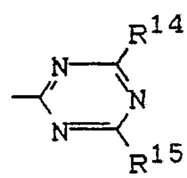

steht, worin
$R^{14}$ und $R^{15}$ gleich oder verschieden sind und für Fluor, Chlor, Brom, Hydroxy, $C_1$-$C_4$-Alkyl [welches gegebenenfalls durch Fluor und/oder Chlor substituiert ist], $C_3$-$C_5$-Cycloalkyl, $C_1$-$C_4$-Alkoxy [welches gegebenenfalls durch Fluor und/oder Chlor substituiert ist], $C_1$-$C_4$-Alkylthio, $C_1$-$C_4$-Alkylamino oder Di-($C_1$-$C_4$-alkyl)-amino stehen.

Verwendet man beispielsweise N-(2-Chlor-benzolsulfonyl)-iminodithiokohlensäurediphenylester und 2-Amino-4-methoxy-6-methyl-triazin als Ausgangsstoffe, so kann der Reaktionsablauf nach dem erfindungsgemäßen Verfahren durch folgendes Formelschema wiedergegeben werden:

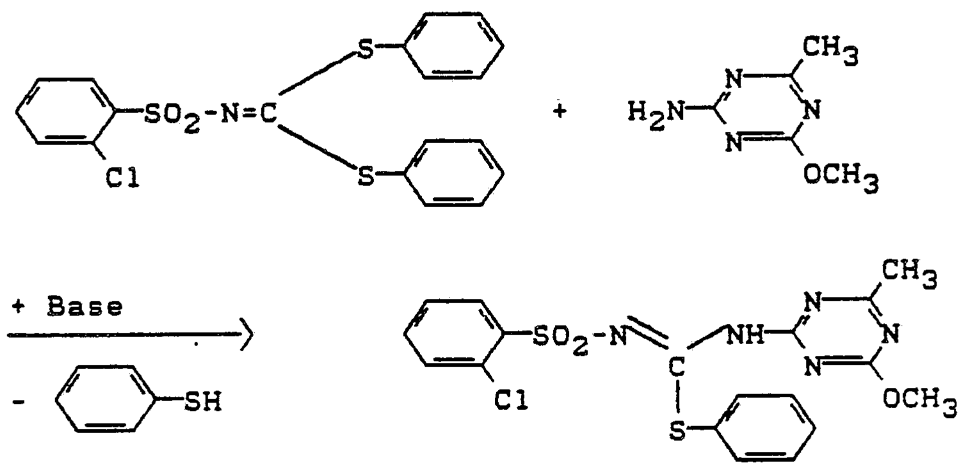


Die beim erfindungsgemäßen Verfahren als Ausgangsstoffe zu verwendenden Iminodithiokohlensäureester sind durch die Formel (II) allgemein definiert. In dieser Formel (II) stehen $R^1$ und $R^2$ vorzugsweise für diejenigen Reste, die bereits im Rahmen der Substituentendefinition der Formel (I) vorzugsweise genannt wurden.

Die Verbindungen der Formel (II) sind teilweise bekannt (vergl. Chem. Ber. 99, 2900 (1960)). Neu und Teil der Erfindung sind die Verbindungen der Formel (IIa)

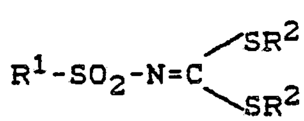
(IIa)

in welcher
$R^1$ für den Rest

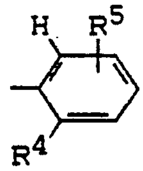

steht, worin

$R^4$ und $R^5$ gleich oder verschieden sind und für Wasserstoff, Halogen [wie insbesondere Fluor, Chlor, Brom und/oder Iod], Cyano, $C_1$-$C_6$-Alkyl, [welches gegebenenfalls durch Fluor, Chlor, Brom, Cyano $C_1$-$C_4$-Alkoxy, $C_3$-$C_6$-Cycloalkyl oder Phenyl substituiert ist], für $C_1$-$C_4$-Alkoxy [welches gegebenenfalls durch Fluor, Chlor, Brom, Cyano oder $C_1$-$C_4$-Alkoxy substituiert ist], für Di-($C_1$-$C_4$-alkyl)-aminosulfonyl, für Phenyl oder Phenoxy, für $C_1$-$C_4$-Alkylcarbonylamino, $C_1$-$C_4$-Alkylamino-carbonyl-amino, Di-($C_1$-$C_4$-alkyl)-amino-carbonylamino, Di-($C_1$-$C_4$-alkyl)-aminocarbonyl oder für Di-($C_1$-$C_4$-alkyl)-aminosulfonylamino stehen, worin weiter

$R^1$ für den Rest

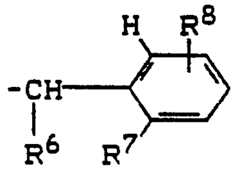

steht, worin

$R^6$ für Wasserstoff oder $C_1$-$C_4$-Alkyl steht,

$R^7$ und $R^8$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist] oder Di-($C_1$-$C_4$-alkyl)-aminosulfonyl stehen; worin weiter

$R^1$ für den Rest

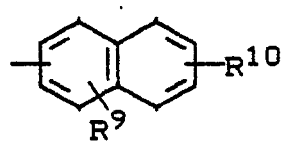

steht, worin

$R^9$ und $R^{10}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist] oder $C_1$-$C_4$-Alkoxy [welches gegebenenfalls durch Fluor und/oder Chlor substituiert ist], stehen; und

$R^2$ für einen Phenylrest steht, welcher gegebenenfalls substituiert ist durch einen oder mehrere Reste aus der Reihe

Halogen [wie insbesondere Fluor, Chlor, Brom und Iod], Cyano, Nitro, Hydroxy, Carboxy, $C_1$-$C_6$-Alkyl [welches gegebenenfalls durch Fluor, Chlor, Brom, Nitro, Cyano, Hydroxy, Carboxy, $C_1$-$C_4$-Alkoxy-carbonyl, $C_1$-$C_4$-Alkoxy, $C_1$-$C_4$-Alkylthio oder Phenyl substituiert ist], $C_3$-$C_6$-Cycloalkyl, $C_1$-$C_4$-Alkoxy [welches gegebenenfalls durch Fluor, Chlor, Brom, Cyano, Carboxy, $C_1$-$C_4$-Alkoxy, $C_1$-$C_4$-Alkylthio oder $C_1$-$C_4$-Alkoxy-carbonyl substituiert ist], $C_1$-$C_4$-Alkylthio [welches gegebenenfalls durch Fluor, Chlor, Brom, Cyano, Carboxy, $C_1$-$C_4$-Alkoxy-carbonyl substituiert ist], Amino, $C_1$-$C_4$-Alkyl-amino bzw. Di-($C_1$-$C_4$-alkyl)-amino [welche gegebenenfalls durch Fluor, Chlor, Brom, Cyano, Carboxy, $C_1$-$C_4$-Alkoxy oder $C_1$-$C_4$-Alkoxy-carbonyl substituiert sind], $C_1$-$C_4$-Alkyl-carbonyl-amino, $C_1$-$C_4$-Alkoxy-carbonyl-amino, (Di)-$C_1$-$C_4$-Alkylamino-carbonyl-amino, Formyl, $C_1$-$C_4$-Alkyl-carbonyl, Benzoyl, $C_1$-$C_4$-Alkoxy-carbonyl, Phenoxy-carbonyl, Benzyloxycarbonyl, Phenyl [welches gegebenenfalls durch Fluor, Chlor, Brom, Cyano, Nitro, Hydroxy, oder Methyl substituiert ist], Phenoxy, Phenylthio, Phenylsulfonyl, Phenylamino oder Phenylazo [welches gegebenenfalls durch Fluor, Chlor, Brom, Cyano, Nitro, Methyl und/oder Trifluormethyl substituiert sind], Pyridoxy oder Pyrimidoxy [welches gegebenenfalls durch Fluor, Chlor, Brom, Cyano, Nitro, Methyl und/oder Trifluormethyl substituiert sind], $C_1$-$C_4$-Alkyl-carbonyloxy, $C_1$-$C_4$-Alkoxy-carbonyloxy, $C_1$-$C_4$-Alkyl-amino-carbonyloxy und Di-($C_1$-$C_4$-alkyl)-amino-carbonyloxy, oder welcher gegebenenfalls durch eine Alkylenkette [welche gegebenenfalls verzweigt und/oder durch ein oder mehrere Sauerstoffatome unterbrochen ist] oder einen Benzorest [welcher gegebenenfalls durch Fluor, Chlor, Brom, Cyano, Nitro, Methyl und/oder Trifluormethyl substituiert ist] anelliert ist; wobei

jedoch die folgenden Verbindungen ausgenommen sind: N-(4-Methyl-benzolsulfonyl)-iminodithiokohlensäurediphenylester und N-(4-Chlor-benzolsulfonyl)-iminodithiokohlensäurediphenylester.

Als Beispiele für die Verbindungen der Formel (IIa) seien genannt:

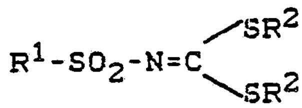 (IIa)

Tabelle 1

| $R^1$ | $R^2$ |
|---|---|
| 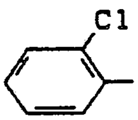 | 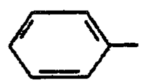 |
| 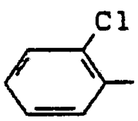 | 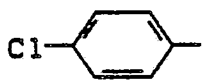 |
| 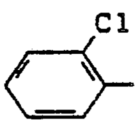 | 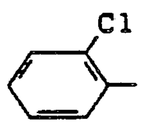 |
| 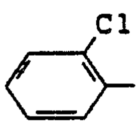 | 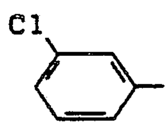 |
| 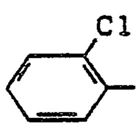 | 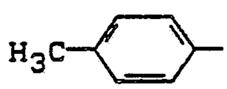 |
| 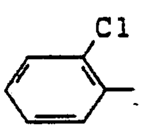 | 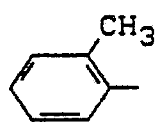 |
| 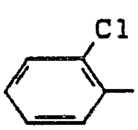 | 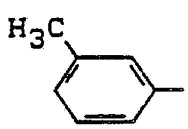 |
| 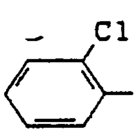 | 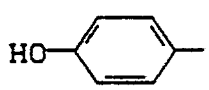 |

Tabelle 1 - Fortsetzung

| $R^1$ | $R^2$ |
|---|---|
| 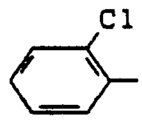 | 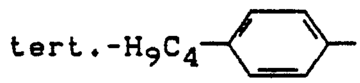 |
| 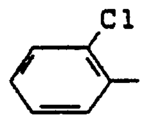 | 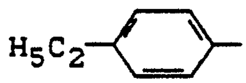 |
| 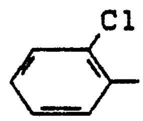 | 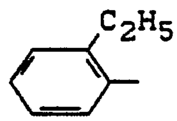 |
| 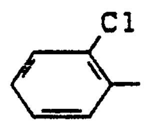 | 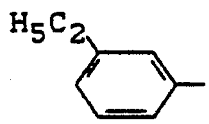 |
| 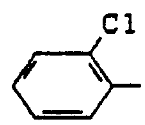 | 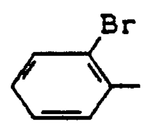 |
| 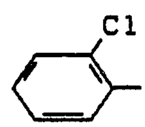 | 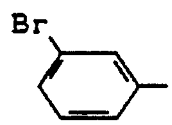 |
| 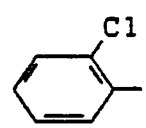 | 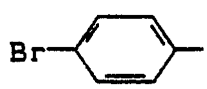 |
| 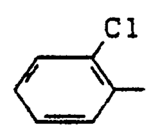 | 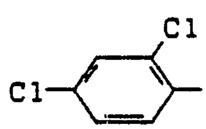 |

<u>Tabelle 1</u> - Fortsetzung

| $R^1$ | $R^2$ |
|---|---|
| 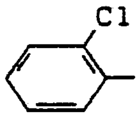 | 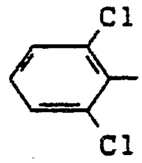 |
| 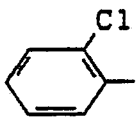 | 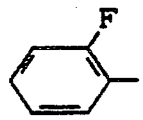 |
| 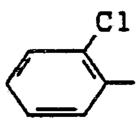 | 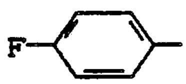 |
| 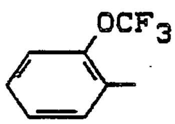 | 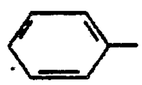 |
| 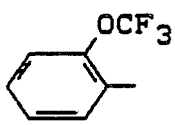 | 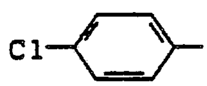 |
| 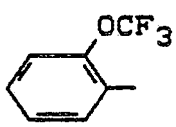 | 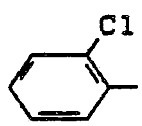 |
| 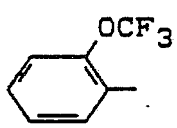 | 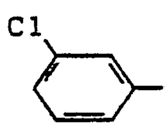 |
| 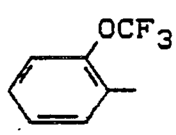 | 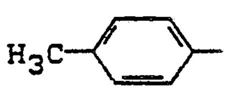 |

Tabelle 1 - Fortsetzung

| $R^1$ | $R^2$ |
|---|---|
| 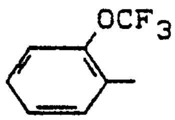 | 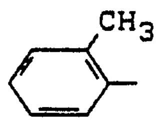 |
| 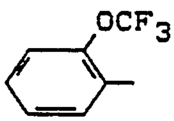 | 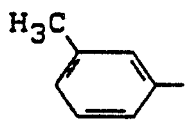 |
| 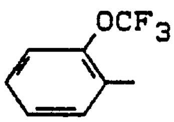 | 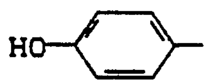 |
| 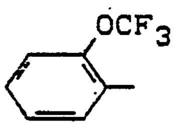 | 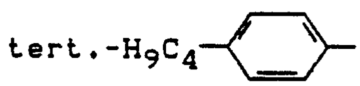 |
| 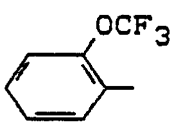 | 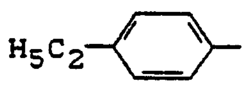 |
| 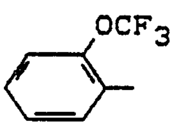 | 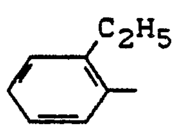 |
| 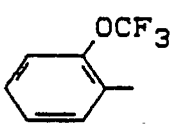 | 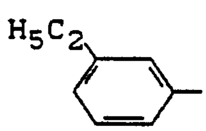 |
| 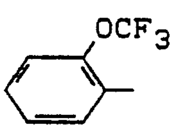 | 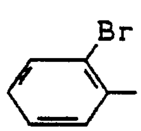 |

Tabelle 1 - Fortsetzung

| $R^1$ | $R^2$ |
|---|---|
| 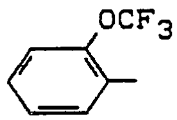 | 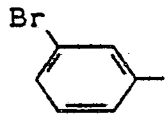 |
| 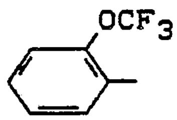 | 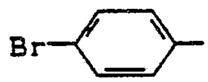 |
| 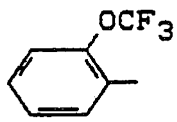 | 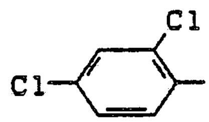 |
| 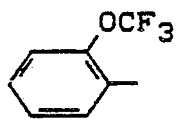 | 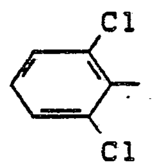 |
| 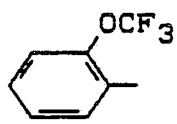 | 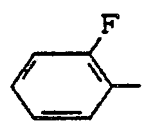 |
| 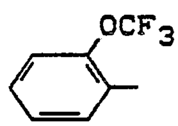 | 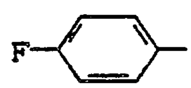 |
| 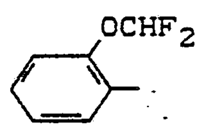 | 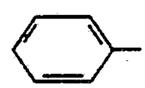 |
| 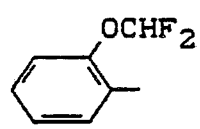 | 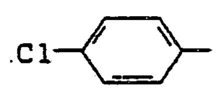 |

Tabelle 1 - Fortsetzung

| $R^1$ | $R^2$ |
| --- | --- |
| 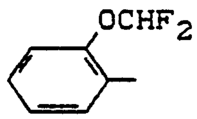 | 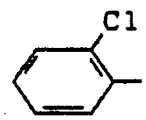 |
| 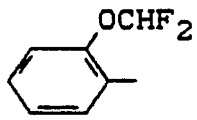 | 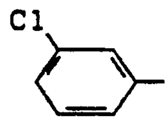 |
| 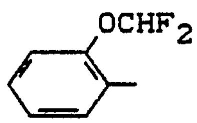 | 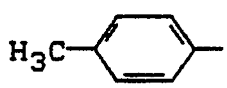 |
| 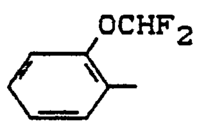 | 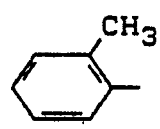 |
| 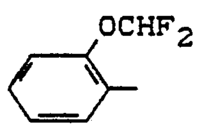 | 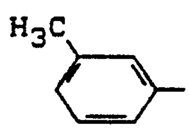 |
| 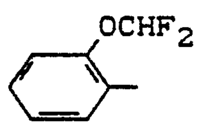 | 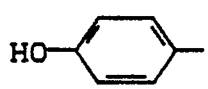 |
| 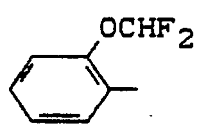 | 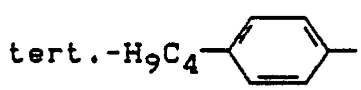 |
| 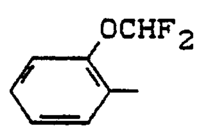 | 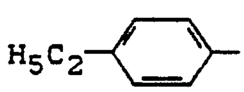 |

Tabelle 1 - Fortsetzung

| $R^1$ | $R^2$ |
|---|---|
| 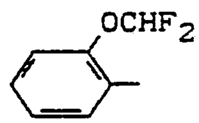 | 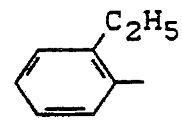 |
| 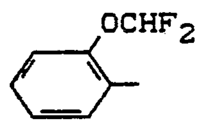 | 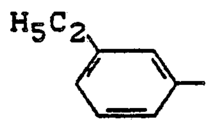 |
| 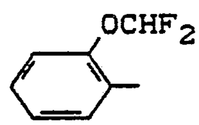 | 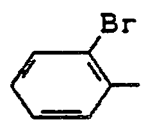 |
| 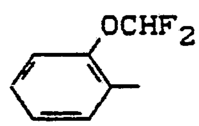 | 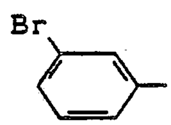 |
| 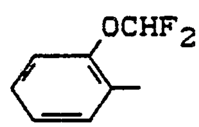 | 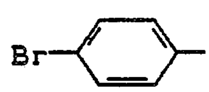 |
| 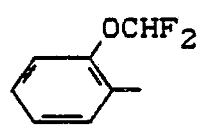 | 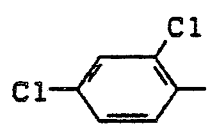 |
| 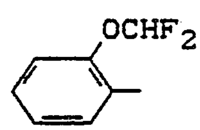 | 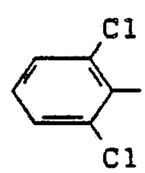 |
| 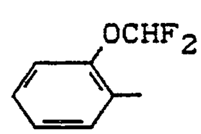 | 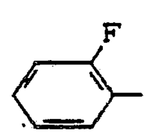 |

Tabelle 1 - Fortsetzung

| $R^1$ | $R^2$ |
|---|---|
| 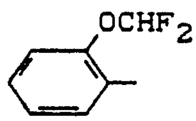 | 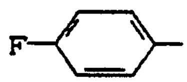 |
| 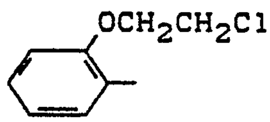 | 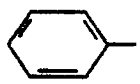 |
| 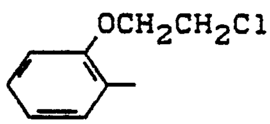 | 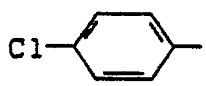 |
| 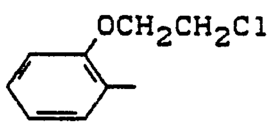 | 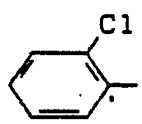 |
| 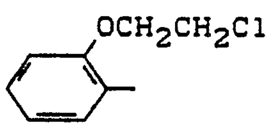 | 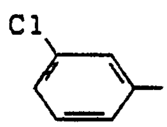 |
| 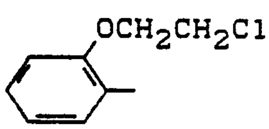 | 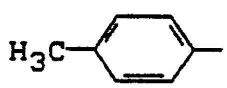 |
| 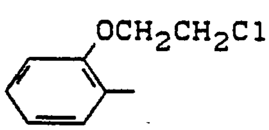 | 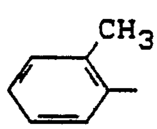 |
| 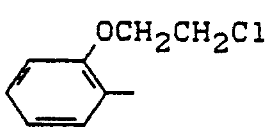 | 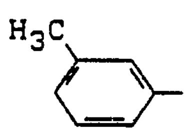 |

<u>Tabelle 1</u> - Fortsetzung

| $R^1$ | $R^2$ |
|---|---|
| 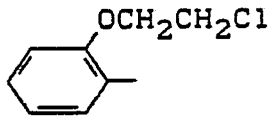 | 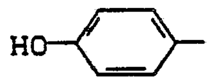 |
| 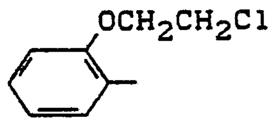 | 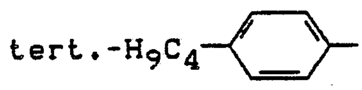 |
| 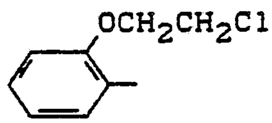 | 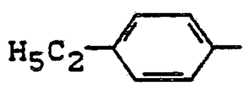 |
| 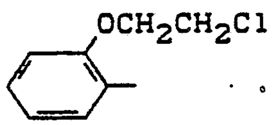 | 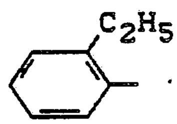 |
| 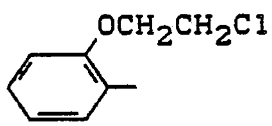 | 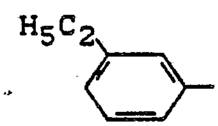 |
| 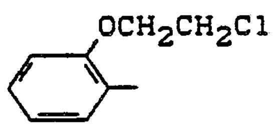 | 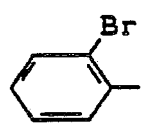 |
| 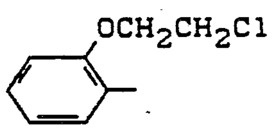 | 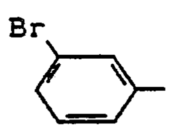 |
| 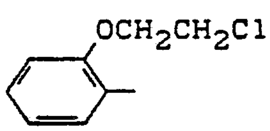 | 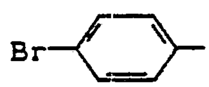 |

Tabelle 1 - Fortsetzung

| $R^1$ | $R^2$ |
|---|---|
| 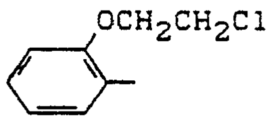 | 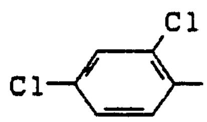 |
| 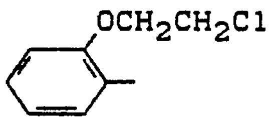 | 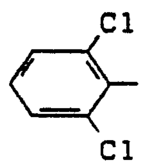 |
| 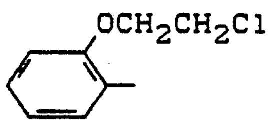 | 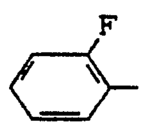 |
| 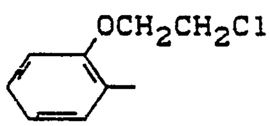 | 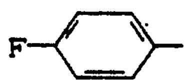 |
| 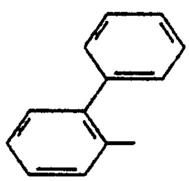 | 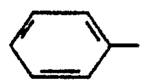 |
| 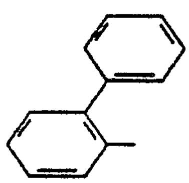 | 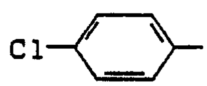 |
| 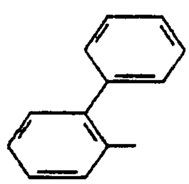 | 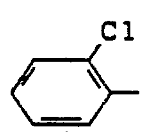 |

Tabelle 1 - Fortsetzung

| $R^1$ | $R^2$ |
|---|---|
| 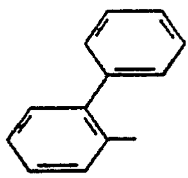 | 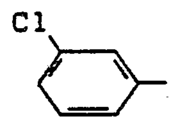 |
| 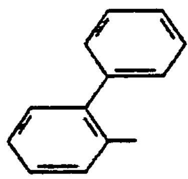 | 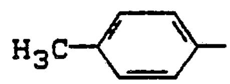 |
| 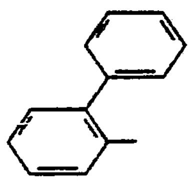 | 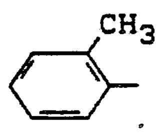 |
| 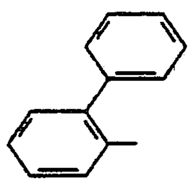 | 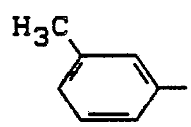 |
| 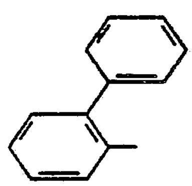 | 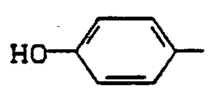 |
| 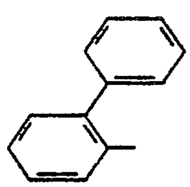 | 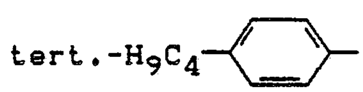 |

<u>Tabelle 1</u> - Fortsetzung

| $R^1$ | $R^2$ |
|---|---|
| 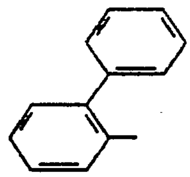 | 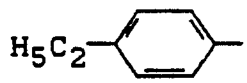 |
| 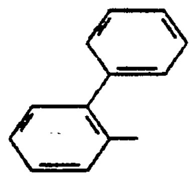 | 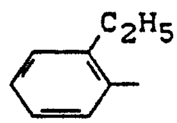 |
| 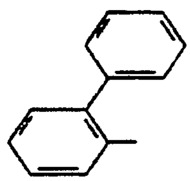 | 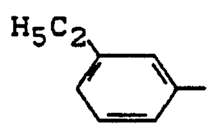 |
| 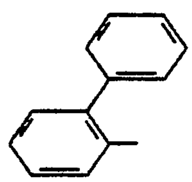 | 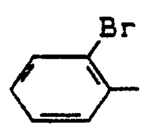 |
| 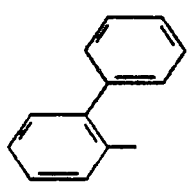 | 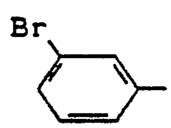 |
| 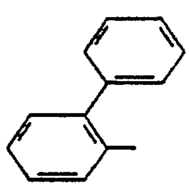 | 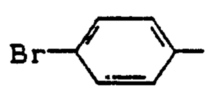 |

<u>Tabelle 1</u> - Fortsetzung

| $R^1$ | $R^2$ |
|---|---|
| 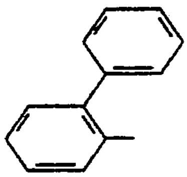 | 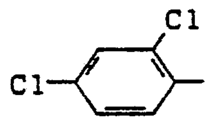 |
| 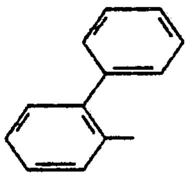 | 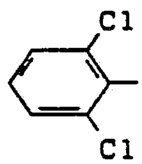 |
| 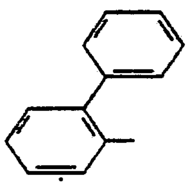 | 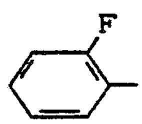 |
| 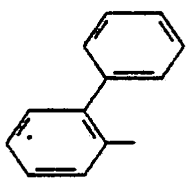 | 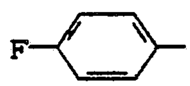 |
| 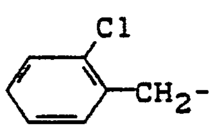 | 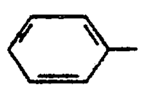 |
| 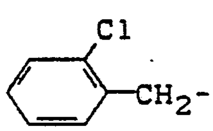 | 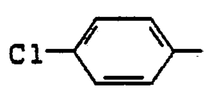 |
| 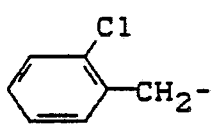 | 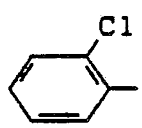 |

Tabelle 1 - Fortsetzung

| $R^1$ | $R^2$ |
|---|---|
| 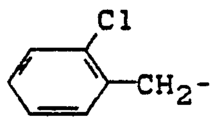 | 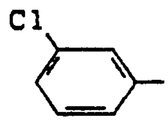 |
| 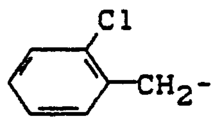 | 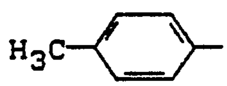 |
| 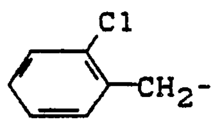 | 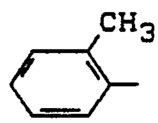 |
| 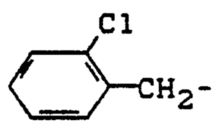 | 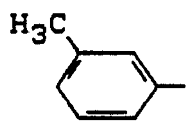 |
| 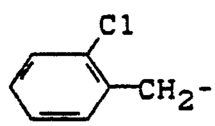 | 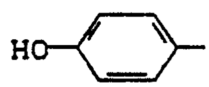 |
| 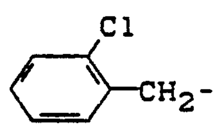 | 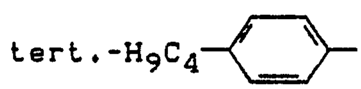 |
| 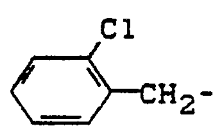 | 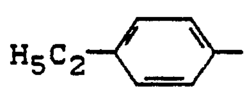 |
| 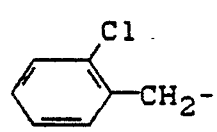 | 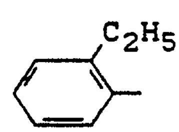 |
| 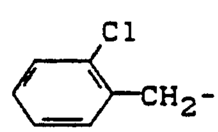 | 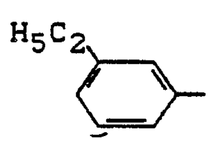 |

<u>Tabelle 1</u> - Fortsetzung

| $R^1$ | $R^2$ |
|---|---|
| 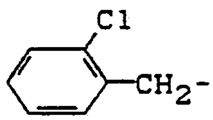 | 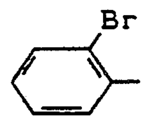 |
| 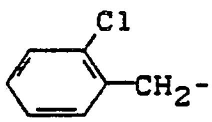 | 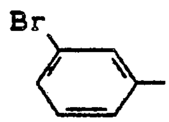 |
| 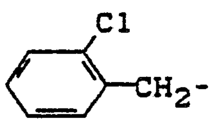 | 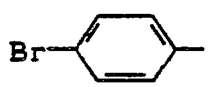 |
| 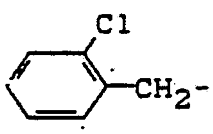 | 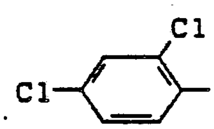 |
| 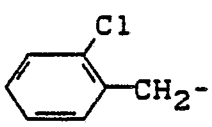 | 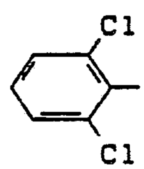 |
| 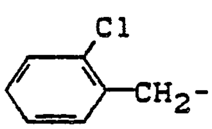 | 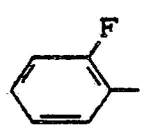 |
| 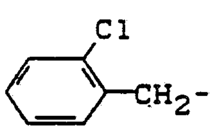 | 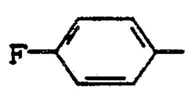 |
| 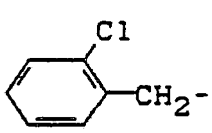 | 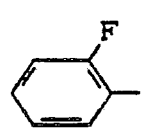 |

<u>Tabelle 1</u> - Fortsetzung

| $R^1$ | $R^2$ |
|---|---|
| 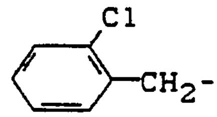 | 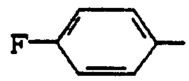 |
| 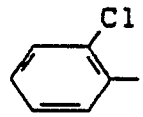 | 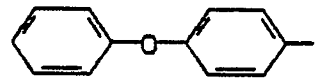 |
| 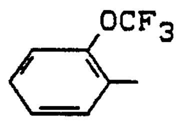 | 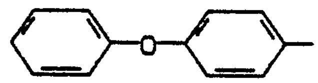 |
| 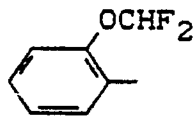 | 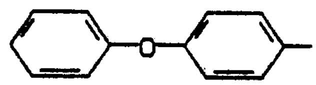 |
| 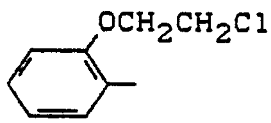 | 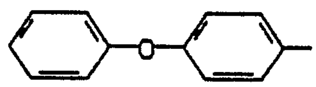 |
| 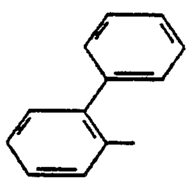 | 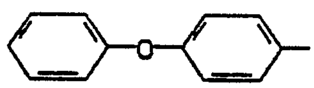 |
| 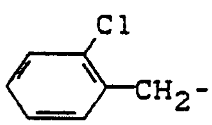 | 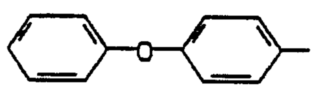 |
| 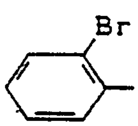 | 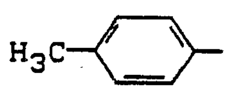 |

Tabelle 1 - Fortsetzung

| $R^1$ | $R^2$ |
|---|---|
| 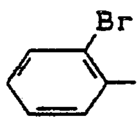 | 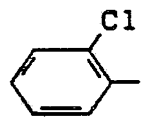 |
| 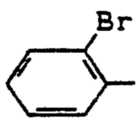 | 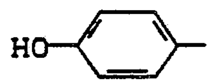 |
| 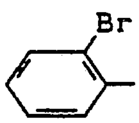 | 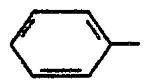 |
| 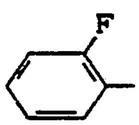 | 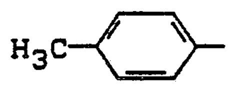 |
| 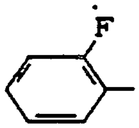 | 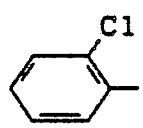 |
| 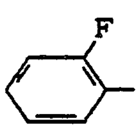 | 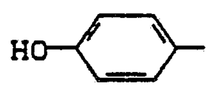 |
| 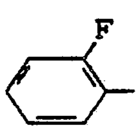 | 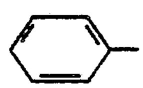 |
| 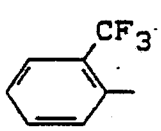 | 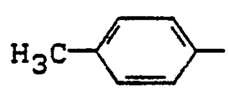 |
| 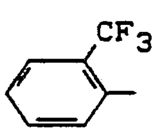 | 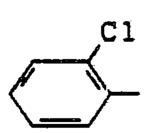 |

Tabelle 1 - Fortsetzung

| $R^1$ | $R^2$ |
|---|---|
| 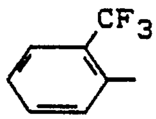 | 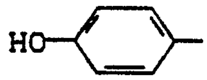 |
| 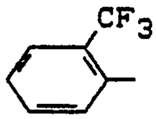 | 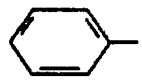 |
| 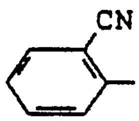 | 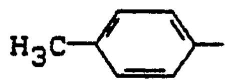 |
| 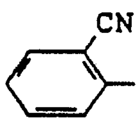 | 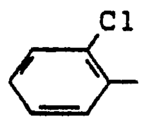 |
| 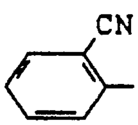 | 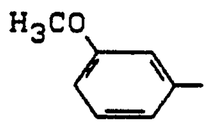 |
| 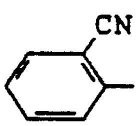 | 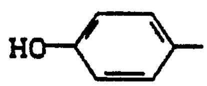 |
| 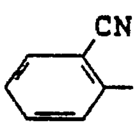 | 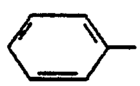 |
| 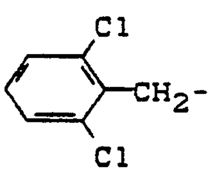 | 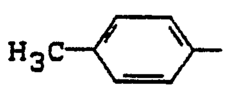 |

Tabelle 1 - Fortsetzung

| $R^1$ | $R^2$ |
|---|---|
| 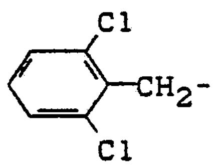 | 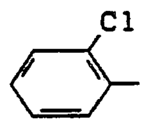 |
| 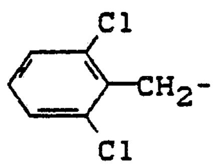 | 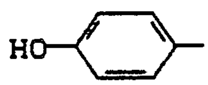 |
| 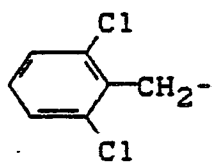 | 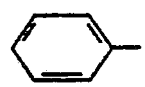 |
| 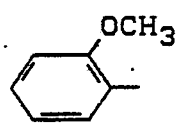 | 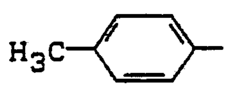 |
| 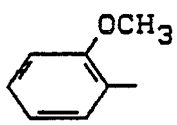 | 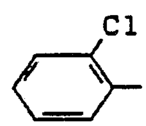 |
| 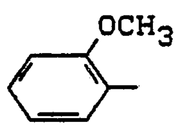 | 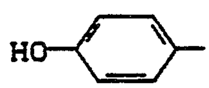 |
| 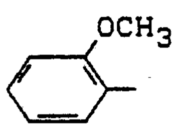 | 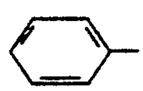 |
| 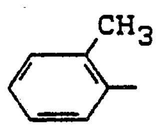 | 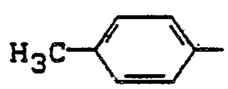 |

Tabelle 1 - Fortsetzung

| $R^1$ | $R^2$ |
|---|---|
| 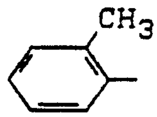 | 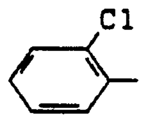 |
| 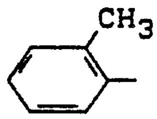 | 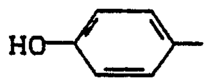 |
| 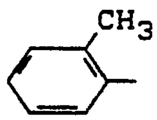 | 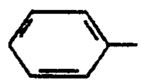 |
| 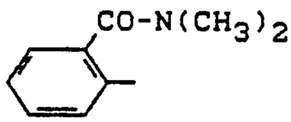 | 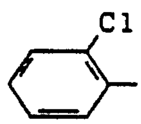 |
| 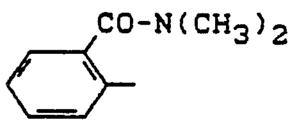 | 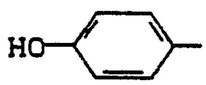 |
| 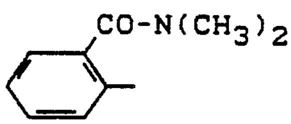 | 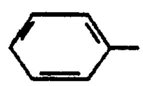 |
| 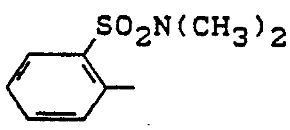 | 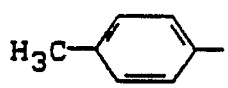 |
| 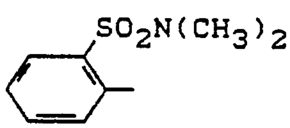 | 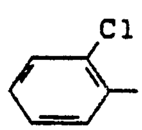 |

<u>Tabelle 1</u> - Fortsetzung

| $R^1$ | $R^2$ |
|---|---|
| 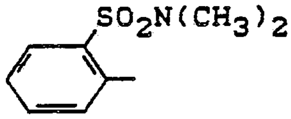 | 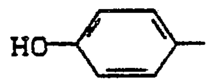 |
| 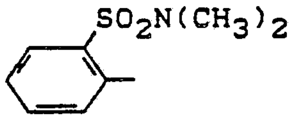 | 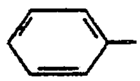 |

Man erhält die Verbindungen der Formel (II) bzw. (IIa), wenn man Iminokohlensäuredichloride der Formel (IV)

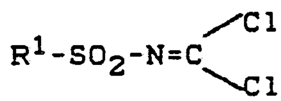 (IV)

in welcher
$R^1$ die oben angegebene Bedeutungen hat,
mit Verbindungen der Formel (V)
$R^2$-SM (V)
in welcher
$R^2$ die oben angegebenen Bedeutungen hat und
M für Wasserstoff oder ein Alkalimetallatom steht,
gegebenenfalls in Gegenwart von Säureakzeptoren, wie z. B. Natrium-oder Kaliumhydroxid und gegebenenfalls in Gegenwart von Verdünnungsmitteln, wie z. B. Aceton, Methylethylketon oder Acetonitril bei Temperaturen zwischen 10 °C und 100 °C umsetzt.

Die als Ausgangsstoffe für die Herstellung der Verbindungen der Formel (II) bzw. (IIa) einzusetzenden Iminokohlensäuredichloride sind durch die Formel (IV) allgemein definiert. In dieser Formel steht $R^1$ vorzugsweise für diejenigen Reste, die bereits im Rahmen der Substituentendefinition der Formel (I) vorzugsweise genannt wurden.

Die Iminokohlensäuredichloride der Formel (IV) sind zum Teil bekannt und/oder lassen sich nach bekannten Verfahren herstellen (vergl. z. B. Chem. Ber. <u>99</u>, 2900 (1966), CA 90:137826b; Angew. Chem. <u>77</u>, 430 (1965)). Neu sind die Iminokohlensäuredichloride der Formel (IVa)

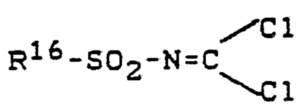 (IVa)

in welcher
$R^{16}$ für den Rest

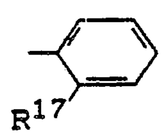

steht, worin
$R^{17}$ für Halogen [wie insbesondere Fluor, Chlor, Brom und/oder Iod], Cyano, $C_2$-$C_6$-Alkyl, $C_1$-$C_6$-Alkyl [welches durch Fluor, Chlor, Brom, Cyano, $C_1$-$C_4$-Alkoxy, $C_3$-$C_6$-Cycloalkyl oder Phenyl substituiert ist], für $C_1$-$C_4$-Alkoxy [welches gegebenenfalls durch Fluor, Chlor, Brom, Cyano oder $C_1$-$C_4$-Alkoxy substituiert ist],

für Di-($C_1$-$C_4$-alkyl)-aminosulfonyl, für Di-($C_1$-$C_4$-alkyl)-aminocarbonyl, für Phenyl oder Phenoxy, für $C_1$-$C_4$-Alkylcarbonylamino, $C_1$-$C_4$-Alkylamino-carbonylamino, Di-($C_1$-$C_4$-alkyl)-amino-carbonylamino, Di-($C_1$-$C_4$-alkyl)-aminocarbonyl oder für Di-($C_1$-$C_4$-alkyl)-aminosulfonylamino steht, worin weiter
$R^{16}$ für den Rest

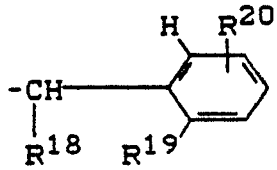

steht, worin
$R^{18}$ für Wasserstoff oder $C_1$-$C_4$-Alkyl steht,
$R^{19}$ und $R^{20}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist] oder Di-($C_1$-$C_4$-alkyl)-aminosulfonyl stehen; worin weiter
$R^{16}$ für den Rest

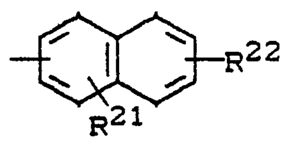

steht, worin
$R^{21}$ und $R^{22}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist] oder $C_1$-$C_4$-Alkoxy [welches gegebenenfalls durch Fluor und/oder Chlor substituiert ist], stehen.

Man erhält die neuen Verbindungen der Formel (IVa), wenn man Iminodithiokohlensäuredimethylester der Formel (VI)

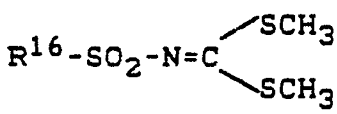 (VI)

in welcher
$R^{16}$ die oben bei Formel (IVa) angegebenen Bedeutungen hat,
in Gegenwart von inerten Verdünnungsmitteln, wie z. B. Tetrachlorkohlenstoff, bei Temperaturen zwischen 0 °C und 25 °C mit einem Chlorierungsmittel, wie z.B. Sulfurylchlorid oder Chlor, umsetzt.

Die Iminodithiokohlensäuredimethylester der Formel (VI) sind bekannt und/oder lassen sich nach bekannten Methoden herstellen (vergl. z. B. EP-A 121 082, EP-A 151 554 und EP-A 173 957).

Als Beispiele für die neuen Iminokohlensäuredichloride der Formel (IVa) seien genannt:

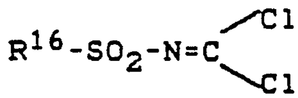 (IVa)

Tabelle 2

| $R^{16}$ | $R^{16}$ |
|---|---|
| 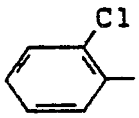 | 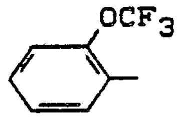 |
| 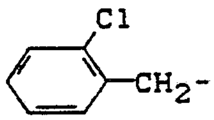 | 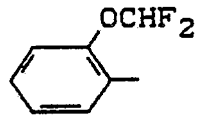 |
| 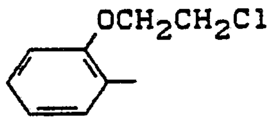 | 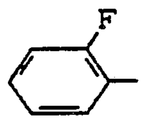 |
| 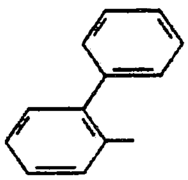 | 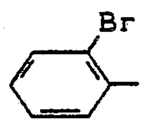 |
| 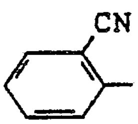 | 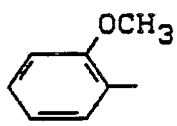 |
| 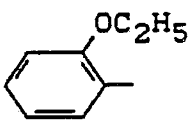 | 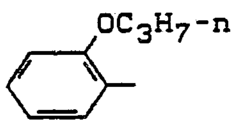 |
| 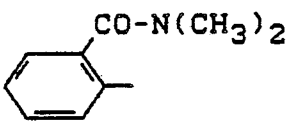 | 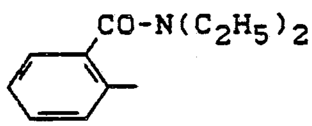 |
| 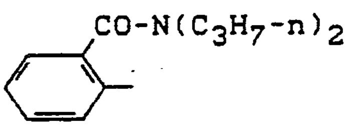 | |

Die weiterhin als Ausgangsstoffe für die Herstellung der Verbindungen der Formel (II) einzusetzenden Verbindungen sind durch die Formel (V) allgemein definiert. In dieser Formel steht $R^2$ vorzugsweise für diejenigen Reste, die bereits im Rahmen der Substituentendefinition der Formel (I) vorzugsweise genannt wurden.

Die Verbindungen der Formel (V) sind allgemein bekannte Verbindungen der organischen Chemie.

Die beim erfindungsgemäßen Verfahren außerdem als Ausgangsstoffe zu verwendenden Amine sind durch die Formel (III) allgemein definiert. In dieser Formel (III) steht $R^3$ vorzugsweise für diejenigen Reste, die bereits im Rahmen der Substituentendefinition der Formel (I) vorzugsweise genannt wurden.

Als Beispiele für die Amine der Formel (III) seien genannt:

2-Amino-pyrimidin sowie 4-Ethyl-6-methyl-, 4-Ethoxy-6-methoxy-, 4,6-Dimethyl-, 4,6-Diethyl-, 4-Methoxy-6-methylthio-, 4-Chlor-6-ethyl-, 5-Chlor-4,6-dimethyl-, 4-Methyl-, 4-Ethyl-, 4-Methoxy-6-methyl-, 4-Ethoxy-6-methyl-, 4,6-Diethoxy-, 5-Ethoxycarbonyl-4-methyl-, 4-Hydroxy-6-methyl-, 4-Chlor-6-methyl-, 4-Methoxy-6-dimethylamino-, 4-Chlor-6-methoxy-, 4-Chlor-6-ethoxy-, 4-Chlor-6-dimethylamino-, 4,5-Dimethyl-, 4-Ethyl-6-methoxy-, 4-Ethoxy-6-ethyl-, 4-Methyl-6-methylthio-, 4,6-Dimethoxy-, 4-Difluormethoxy-6-methyl-, 4-Methoxy-6-methylamino-und 4-Dimethylamino-6-methyl-2-aminopyrimidin;

sowie 4,6-Dimethyl-, 4-Methoxy-6-methyl-, 4-Ethoxy-6-methyl-, 4,6-Dimethoxy-, 4,6-Diethoxy-, 4-Ethoxy-6-methoxy-, 4-Methyl-6-methylthio-, 4-Ethylthio-6-methyl-, 4-Methoxy-6-methylthio-, 4-Ethoxy-6-methylthio-, 4-Ethylthio-6-methoxy-, 4-Ethoxy-6-ethylthio-, 4,6-Dimethylthio-, 4,6-Diethylthio-, 4-Methyl-6-methylamino-, 4-Ethylamino-6-methyl-, 4-Dimethylamino-6-methyl-, 4-Diethylamino-6-methyl-, 4-Methoxy-6-methylamino-, 4-Ethylamino-6-methoxy-, 4-Dimethylamino-6-methoxy-, 4-Diethylamino-6-methoxy-, 4-Ethoxy-6-methylamino-, 4-Ethoxy-6-ethylamino-, 4-Dimethylamino-6-ethoxy-, 4-Diethylamino-6-ethoxy-, 4-Methylamino-6-methylthio-, 4-Ethylamino-6-methylthio-, 4-Dimethylamino-6-methylthio-, 4-Diethylamino-6-methylthio-, 4-Ethylthio-6-methylamino-, 4-Dimethylamino-6-ethylthio-und 4-Diethylamino-6-ethylthio-2-amino-1,3,5-triazin.

Die Amine der Formel (III) sind bekannt und/oder lassen sich nach bekannten Methoden herstellen (vergl. z. B. Chem. Pharm. Bull. 11 (1963), 1382 - 1388; J. Chem. Soc. 1946, 81; US-PS 4 299 960 und EP-A 173 957).

Das erfindungsgemäße Verfahren wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Di-glykoldimethylether, Tetrahydrofuran und Dioxan, Ketone wie Aceton, Methyl-ethyl-, Methyl-isopropyl-und Methyl-isobutyl-keton, Ester wie Essigsäuremethylester und -ethylester, Nitrile wie z. B. Acetonitril und Propionitril, Amide wie z B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren werden bei dem erfindungsgemäßen Verfahren zur Herstellung der Verbindungen der Formel (I) alle üblicherweise für derartige Umsetzungen verwendbaren Säurebindemittel eingesetzt. Vorzugsweise in Frage kommen Alkalimetallhydroxide wie z. B. Natrium-und Kaliumhydroxid, Erdalkalihydroxide wie z. B. Calciumhydroxide, Alkalicarbonate und -alkoholate wie Natrium-und Káliumcarbonat, Natrium-und Kaliummethylat bzw. -ethylat oder Kalium-tert.-butylat.

Die Reaktionen werden im allgemeinen bei Temperaturen zwischen 0 °C und 150 °C, vorzugsweise bei Temperaturen zwischen 10 °C und 100 °C durchgeführt. Die Reaktionen werden im allgemeinen bei Normaldruck durchgeführt.

Zur Durchführung des erfindungsgemäßen Verfahrens werden die Ausgangsstoffe der Formel (II) und (III) und gegebenenfalls der geeignete Säureakzeptor in äquimolaren Mengen eingesetzt.

Bevorzugt werden eine Verbindung der Formel (III), der Säureakzeptor und das Verdünnungsmittel wie z. B. Tetrahydrofuran vorgelegt und einige Stunden gerührt. Anschlies send wird die Verbindung der Formel (II) zugegeben. Nach der Umsetzung wird die Reaktionslösung eingeengt, mit Wasser versetzt, über Kieselgel abgesaugt und mit einer Mineralsäure wie z. B. Salzsäure schwach angesäuert. Die Lösung wird in einem organischen Verdünnungsmittel wie z. B. Methylenchlorid aufgenommen. Die organische Phase wird mit gesättigter Natriumcarbonatlösung und anschließend mit Wasser gewaschen, getrocknet und eingeengt. Die Reinigung der Rohprodukte geschieht nach üblichen Methoden.

Die nach diesem Verfahren leicht zugänglichen Verbindungen der Formel (I) sind bekannt und lassen sich als Pflanzenschutzmittel, insbesondere als Herbizide einsetzen (vergl. z. B. EP-A 173 957).

Herstellungsbeispiele

Beispiel 1

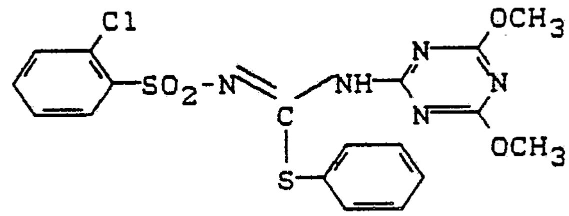

4,5 g (0,0284 Mol) 2-Amino-4,6-dimethoxy-1,3,5-triazin werden in 100 ml Tetrahydrofuran gelöst und mit 3,5 g (0,0284 Mol) Kalium-tert.-butylat (95 %ig) versetzt. Es wird 2 Stunden bei 20 °C gerührt und bei dieser Temperatur werden 11,9 g (0,0284 Mol) 2-Chlorbenzolsulfonyl-imino-kohlensäure-S,S-diphenylester zugegeben. Die Reaktion verläuft schwach exotherm. Es wird 15 Stunden bei 20 °C gerührt, dann auf 50 ml Eiswasser gegossen und abgesaugt. Mit verdünnter 2N Salzsäure wird schwach sauer gestellt und mit Methylenchlorid extrahiert. Die organische Phase wird gewaschen und getrocknet und im Vakuum eingeengt. Der Rückstand wird durch Verreiben mit Methanol fest.

Man erhält so 9,6 g (72,7 % Theorie) an N'-(4,6-Dimethoxy-1,3,5-triazin-2-yl)-N''-(2-chlor-benzolsulfonyl)-S-phenyl-isothioharnstoff vom Schmelzpunkt 149 °C.

Beispiel 2

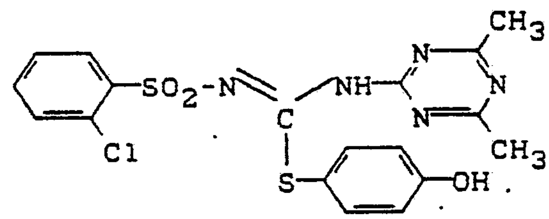

Analog zu Beispiel 1 können beispielsweise auch alle in dem oben genannten Dokument EPA 173 957 beschriebenen Sulfonylisothioharnstoffe hergestellt werden.

Ausgangsverbindungen der Formel (IIa)

Beispiel (IIa-1)

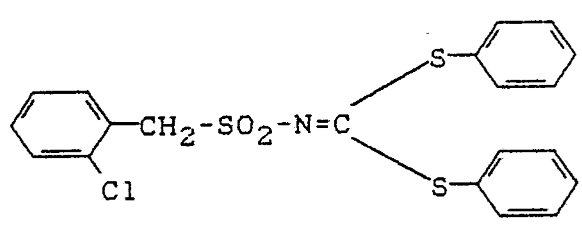

20,7 g (0,073 Mol) N-(2-Chlorbenzylsulfonyl)-iminokohlensäuredichlorid in 200 ml Aceton werden bei 20 °C mit 19,3 g (0,146) Mol) Natrium-Thiophenolat versetzt. Nach Abklingen der exothermen Reaktion wird das Reaktionsgemisch bei ca. 35 °C 3 Stunden gerührt. Anschließend wird das Reaktionsgemisch 15 Stunden bei 20 °C gerührt, filtriert und im Vakuum eingeengt. Der Rückstand wird aus Isopropanol umkristallisiert.

Man erhält so 26 g (85 % der Theorie) N-(2-Chlor-benzylsulfonyl)-iminodithiokohlensäurediphenylester vom Schmelzpunkt 147 °C.

Analog Beispiel (IIa-1) können die folgenden Verbindungen der Formel (IIa) hergestellt werden:

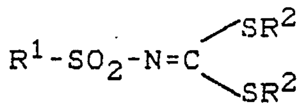 (IIa)

Tabelle 3

| Beisp.-Nr. | $R^1$ | $R^2$ | Physikal. Konstante |
|---|---|---|---|
| IIa-2 | 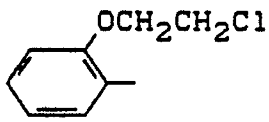 | 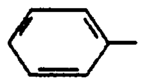 | |
| IIa-3 | 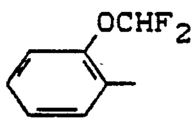 | 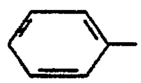 | |
| IIa-4 | 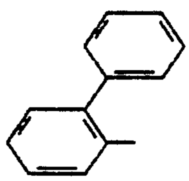 | 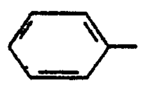 | |
| IIa-5 | 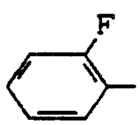 | 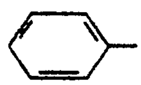 | |
| IIa-6 | 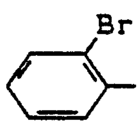 | 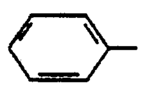 | |
| IIa-7 | 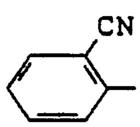 | 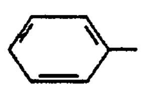 | |
| IIa-8 | 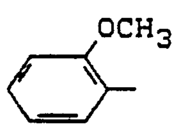 | 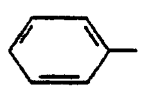 | |
| IIa-9 | 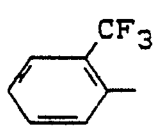 | 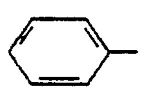 | |

<u>Tabelle 3</u> - Fortsetzung

| Beisp.-Nr. | $R^1$ | $R^2$ | Physikal. Konstante |
|---|---|---|---|
| IIa-10 | 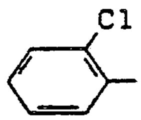 | 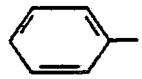 | Fp. 110 °C |
| IIa-11 | 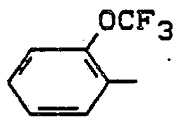 | 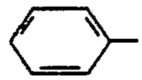 | |
| IIa-12 | 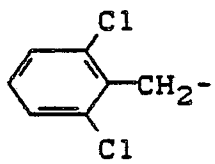 | 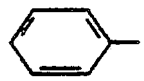 | Fp. 113 °C |
| IIa-13 | 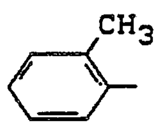 | 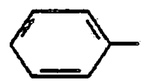 | |
| IIa-14 | 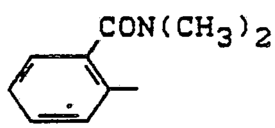 | 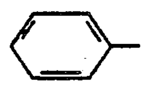 | |
| IIa-15 | 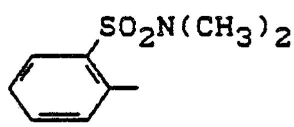 | 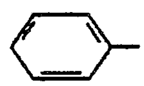 | |
| IIa-16 | 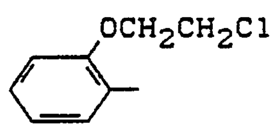 | 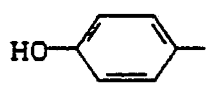 | |
| IIa-17 | 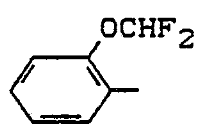 | 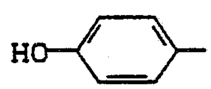 | |

<u>Tabelle 3</u> - Fortsetzung

| Beisp.-Nr. | $R^1$ | $R^2$ | Physikal. Konstante |
|---|---|---|---|
| IIa-18 | 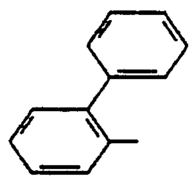 | 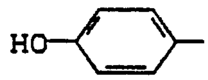 | |
| IIa-19 | 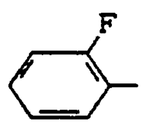 | 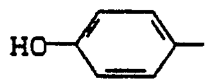 | |
| IIa-20 | 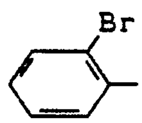 | 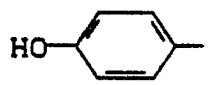 | |
| IIa-21 | 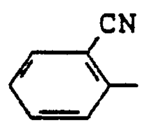 | 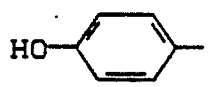 | |
| IIa-22 | 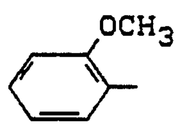 | 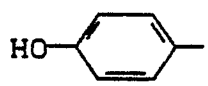 | |
| IIa-23 | 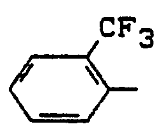 | 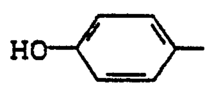 | |
| IIa-24 | 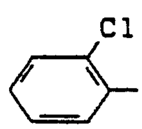 | 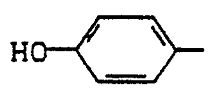 | |
| IIa-25 | 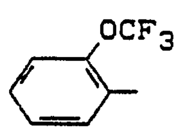 | 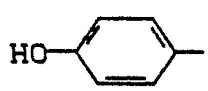 | |

Tabelle 3 - Fortsetzung

| Beisp.-Nr. | $R^1$ | $R^2$ | Physikal. Konstante |
|---|---|---|---|
| IIa-26 | 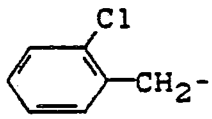 | 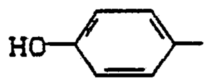 | |
| IIa-27 | 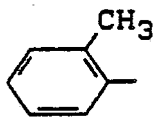 | 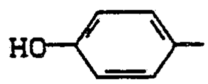 | |
| IIa-28 | 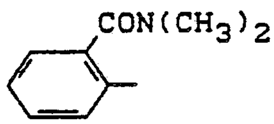 | 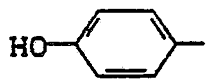 | |
| IIa-29 | 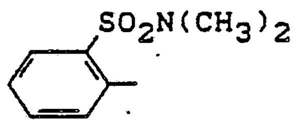 | 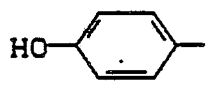 | |
| IIa-30 | 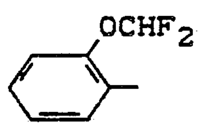 | 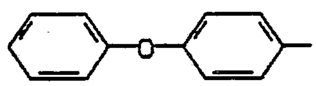 | |
| IIa-31 | 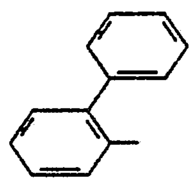 | 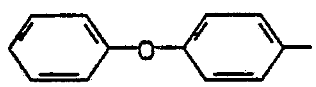 | |
| IIa-32 | 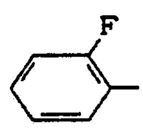 | 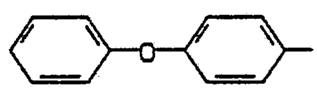 | |
| IIa-33 | 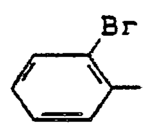 | 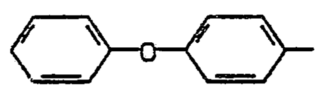 | |

<u>Tabelle 3</u> - Fortsetzung

| Beisp.-Nr. | $R^1$ | $R^2$ | Physikal. Konstante |
|---|---|---|---|
| IIa-34 | 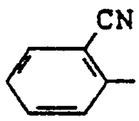 | 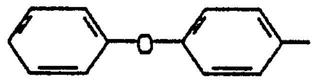 | |
| IIa-35 | 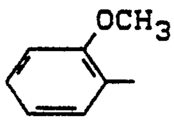 | 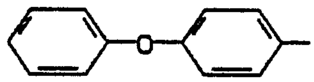 | |
| IIa-36 | 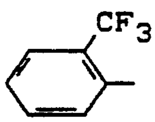 | 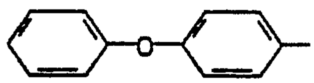 | |
| IIa-37 | 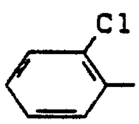 | 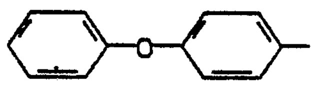 | |
| IIa-38 | 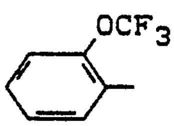 | 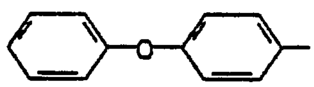 | |
| IIa-39 | 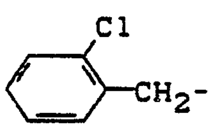 | 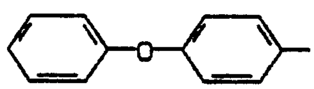 | |
| IIa-40 | 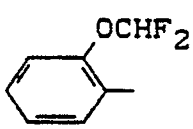 | 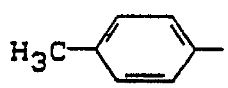 | |
| IIa-41 | 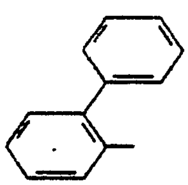 | 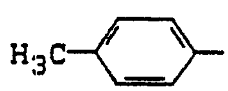 | |

Tabelle 3 - Fortsetzung

| Beisp.-Nr. | $R^1$ | $R^2$ | Physikal. Konstante |
|---|---|---|---|
| IIa-42 | 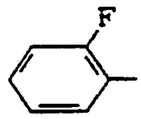 | 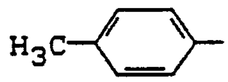 | |
| IIa-43 | 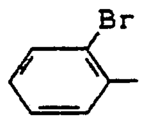 | 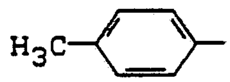 | |
| IIa-44 | 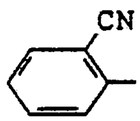 | 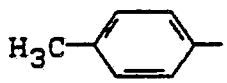 | |
| IIa-45 | 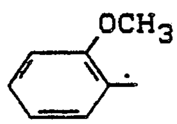 | 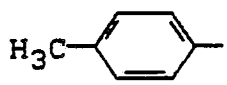 | |
| IIa-46 | 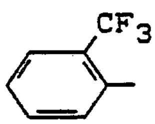 | 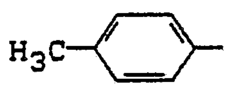 | |
| IIa-47 | 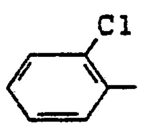 | 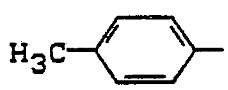 | |
| IIa-48 | 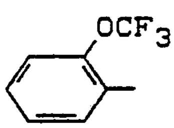 | 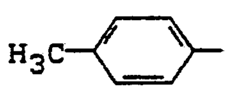 | |
| IIa-49 | 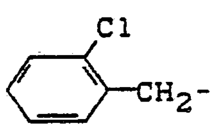 | 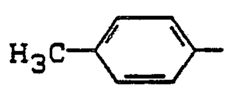 | |

<u>Tabelle 3</u> - Fortsetzung

| Beisp.-Nr. | $R^1$ | $R^2$ | Physikal. Konstante |
|---|---|---|---|
| IIa-50 | 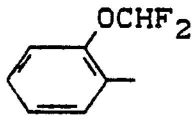 | 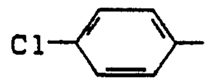 | |
| IIa-51 | 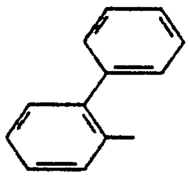 | 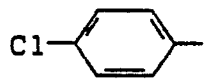 | |
| IIa-52 | 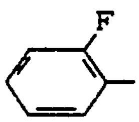 | 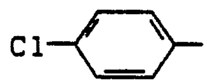 | |
| IIa-53 | 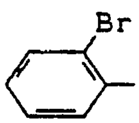 | 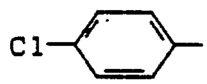 | |
| IIa-54 | 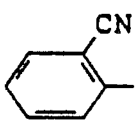 | 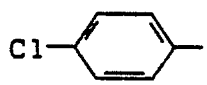 | |
| IIa-55 | 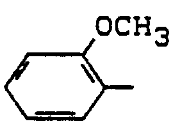 | 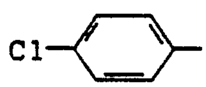 | |
| IIa-56 | 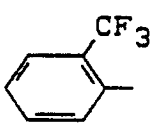 | 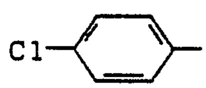 | |
| IIa-57 | 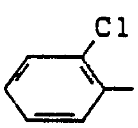 | 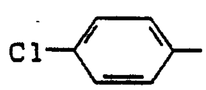 | |

Tabelle 3 - Fortsetzung

| Beisp.-Nr. | $R^1$ | $R^2$ | Physikal. Konstante |
|---|---|---|---|
| IIa-58 | 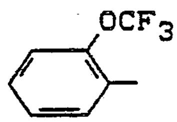 | 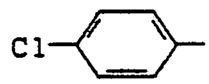 | |
| IIa-59 | 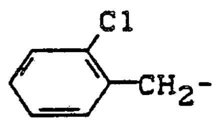 | 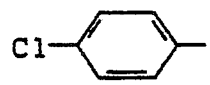 | |
| IIa-60 | 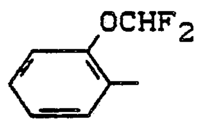 | 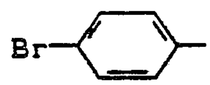 | |
| IIa-61 | 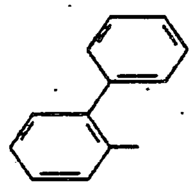 | 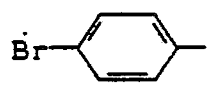 | |
| IIa-62 | 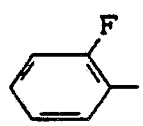 | 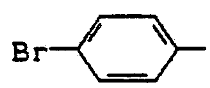 | |
| IIa-63 | 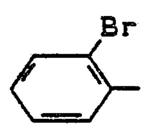 | 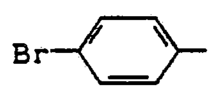 | |
| IIa-64 | 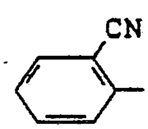 | 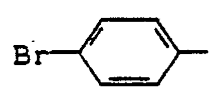 | |
| IIa-65 | 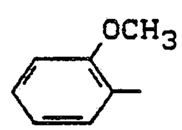 | 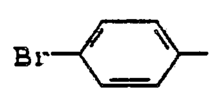 | |

Tabelle 3 - Fortsetzung

| Beisp.-Nr. | $R^1$ | $R^2$ | Physikal. Konstante |
|---|---|---|---|
| IIa-66 | 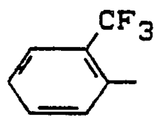 | 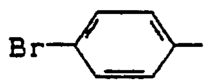 | |
| IIa-67 | 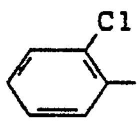 | 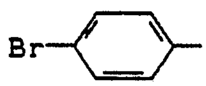 | |
| IIa-68 | 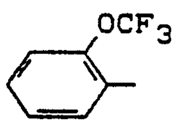 | 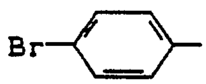 | |
| IIa-69 | 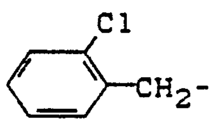 | 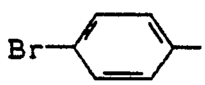 | |
| IIa-70 | 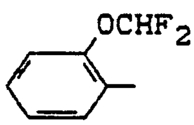 | 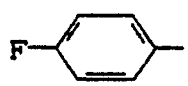 | |
| IIa-71 | 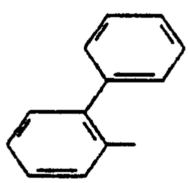 | 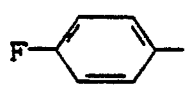 | |
| IIa-72 | 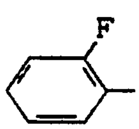 | 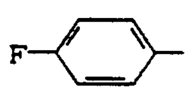 | |
| IIa-73 | 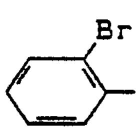 | 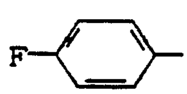 | |

Tabelle 3 - Fortsetzung

| Beisp.-Nr. | $R^1$ | $R^2$ | Physikal. Konstante |
|---|---|---|---|
| IIa-74 | 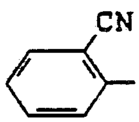 | 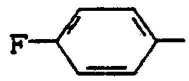 | |
| IIa-75 | 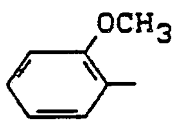 | 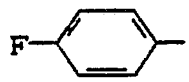 | |
| IIa-76 | 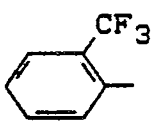 | 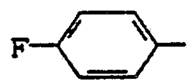 | |
| IIa-77 | 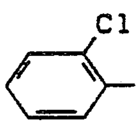 | 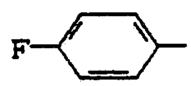 | |
| IIa-78 | 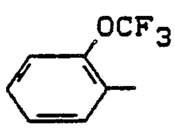 | 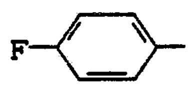 | |
| IIa-79 | 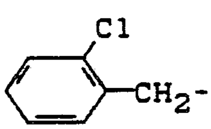 | 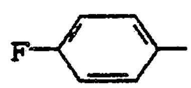 | |
| IIa-80 | 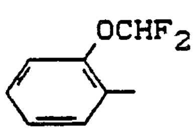 | 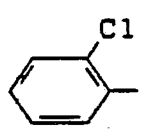 | |
| IIa-81 | 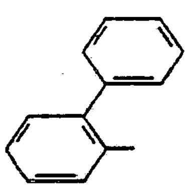 | 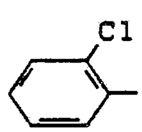 | |

Tabelle 3 - Fortsetzung

| Beisp.-Nr. | $R^1$ | $R^2$ | Physikal. Konstante |
|---|---|---|---|
| IIa-82 | 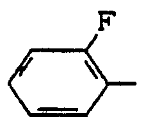 | 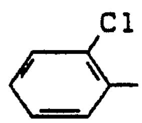 | |
| IIa-83 | 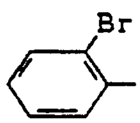 | 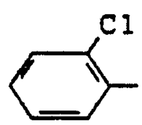 | |
| IIa-84 | 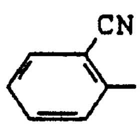 | 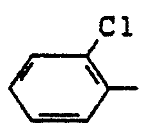 | |
| IIa-85 | 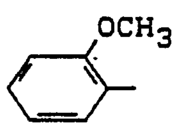 | 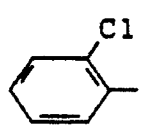 | |
| IIa-86 | 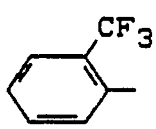 | 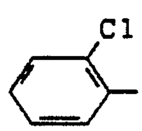 | |
| IIa-87 | 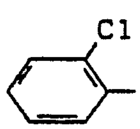 | 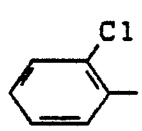 | |
| IIa-88 | 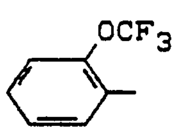 | 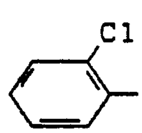 | |
| IIa-89 | 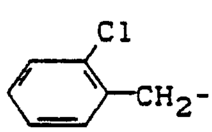 | 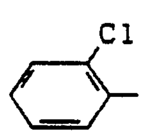 | |

Tabelle 3 - Fortsetzung

| Beisp.-Nr. | $R^1$ | $R^2$ | Physikal. Konstante |
|---|---|---|---|
| IIa-90 | 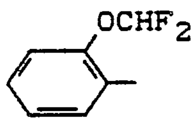 | 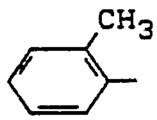 | |
| IIa-91 | 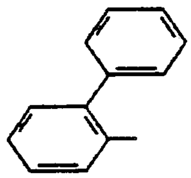 | 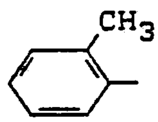 | |
| IIa-92 | 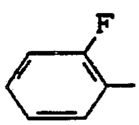 | 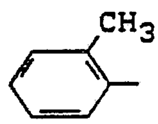 | |
| IIa-93 | 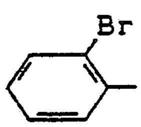 | 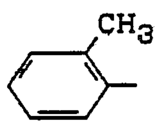 | |
| IIa-94 | 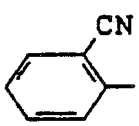 | 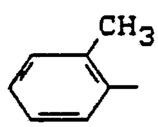 | |
| IIa-95 | 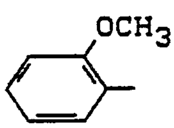 | 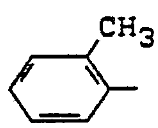 | |
| IIa-96 | 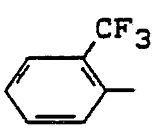 | 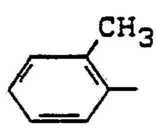 | |
| IIa-97 | 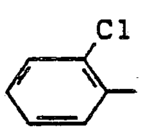 | 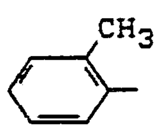 | |

Tabelle 3 - Fortsetzung

| Beisp.-Nr. | $R^1$ | $R^2$ | Physikal. Konstante |
|---|---|---|---|
| IIa-98 | 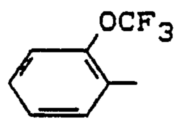 | 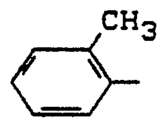 | |
| IIa-99 | 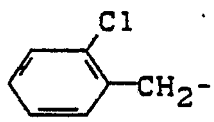 | 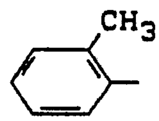 | |
| IIa-100 | 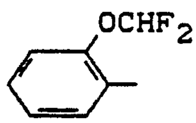 | 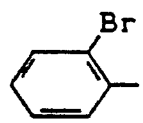 | |
| IIa-101 | 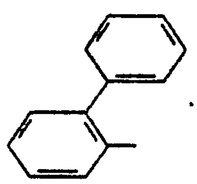 | 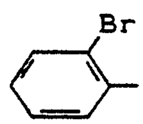 | |
| IIa-102 | 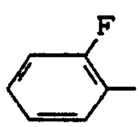 | 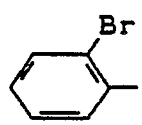 | |
| IIa-103 | 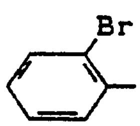 | 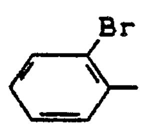 | |
| IIa-104 | 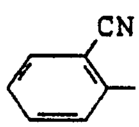 | 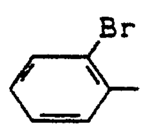 | |
| IIa-105 | 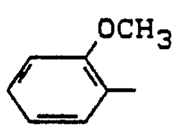 | 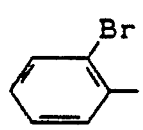 | |

Tabelle 3 - Fortsetzung

| Beisp.-Nr. | $R^1$ | $R^2$ | Physikal. Konstante |
| --- | --- | --- | --- |
| IIa-106 | 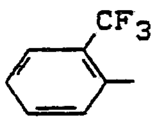 | 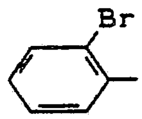 | |
| IIa-107 | 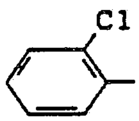 | 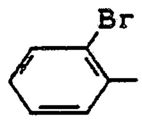 | |
| IIa-108 | 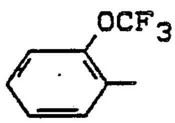 | 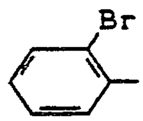 | |
| IIa-109 | 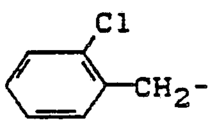 | 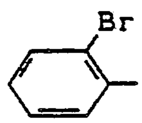 | |
| IIa-110 | 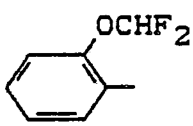 | 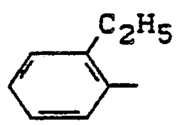 | |
| IIa-111 | 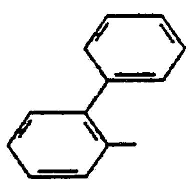 | 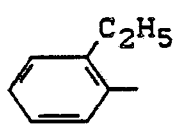 | |
| IIa-112 | 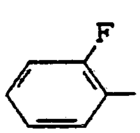 | 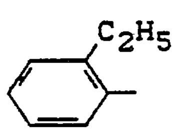 | |
| IIa-113 | 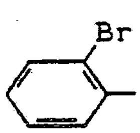 | 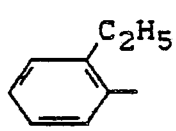 | |

<u>Tabelle 3</u> - Fortsetzung

| Beisp.-Nr. | $R^1$ | $R^2$ | Physikal. Konstante |
|---|---|---|---|
| IIa-114 | 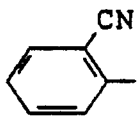 | 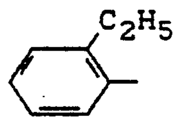 | |
| IIa-115 | 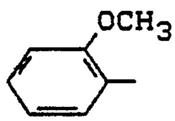 | 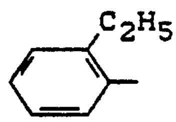 | |
| IIa-116 | 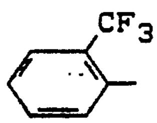 | 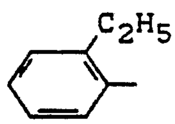 | |
| IIa-117 | 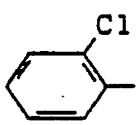 | 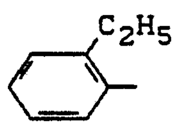 | |
| IIa-118 | 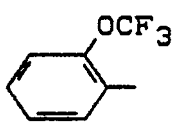 | 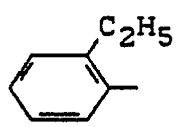 | |
| IIa-119 | 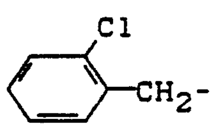 | 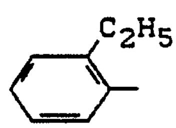 | |
| IIa-120 | 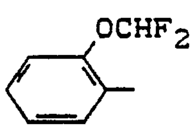 | 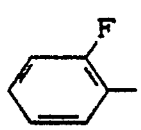 | |
| IIa-121 | 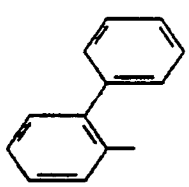 | 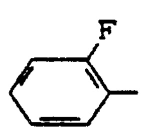 | |

<u>Tabelle 3</u> - Fortsetzung

| Beisp.-Nr. | $R^1$ | $R^2$ | Physikal. Konstante |
|---|---|---|---|
| IIa-122 | 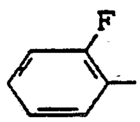 | 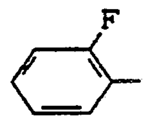 | |
| IIa-123 | 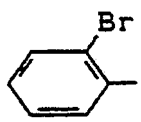 | 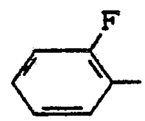 | |
| IIa-124 | 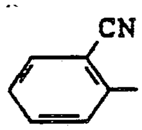 | 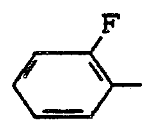 | |
| IIa-125 | 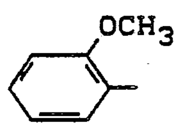 | 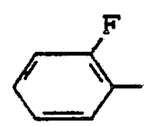 | |
| IIa-126 | 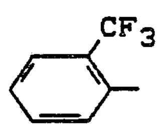 | 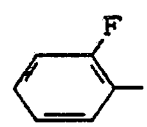 | |
| IIa-127 | 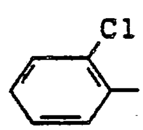 | 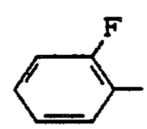 | |
| IIa-128 | 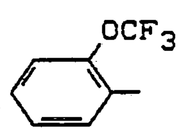 | 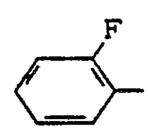 | |
| IIa-129 | 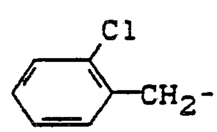 | 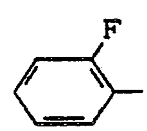 | |

<u>Tabelle 3</u> - Fortsetzung

| Beisp.-Nr. | $R^1$ | $R^2$ | Physikal. Konstante |
|---|---|---|---|
| IIa-130 | 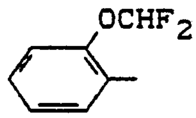 | tert.-$H_9C_4$ 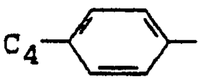 | |
| IIa-131 | 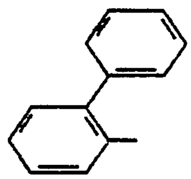 | tert.-$H_9C_4$ 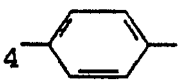 | |
| IIa-132 | 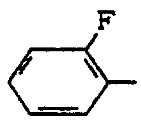 | tert.-$H_9C_4$ 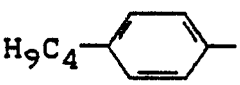 | |
| IIa-133 | 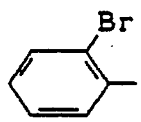 | tert.-$H_9C_4$ 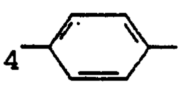 | |
| IIa-134 | 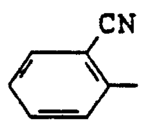 | tert.-$H_9C_4$ 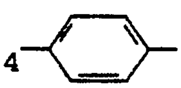 | |
| IIa-135 | 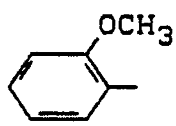 | tert.-$H_9C_4$ 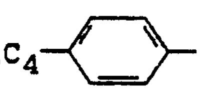 | |
| IIa-136 | 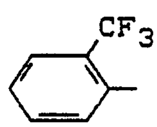 | tert.-$H_9C_4$ 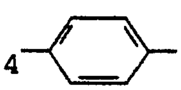 | |
| IIa-137 | 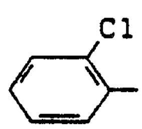 | tert.-$H_9C_4$ 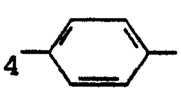 | |

Tabelle 3 - Fortsetzung

| Beisp.-Nr. | $R^1$ | $R^2$ | Physikal. Konstante |
|---|---|---|---|
| IIa-138 | 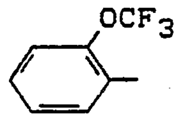 | 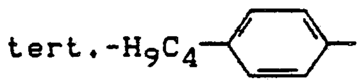 | |
| IIa-139 | 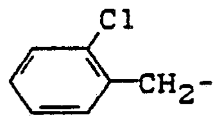 | 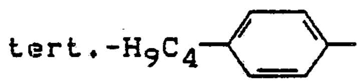 | |

Ausgangsverbindungen der Formel (IV) und (IVa)

Beispiel (IVa-1)

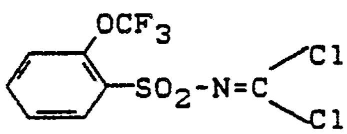

34,5 g (0,1 Mol) 2-Trifluormethoxy-benzolsulfonyl-iminokohlensäure-S,S-dimethylester werden in 300 ml Tetrachlorkohlenstoff gelöst und bei 20 °C werden 568 g (8 Mol) trockenes Chlorgas eingeleitet. Das Reaktionsgemisch erwärmt sich dabei auf etwa 40 °C. Es wird 1 Stunde nachgerührt, abfiltriert, das Filtrat im Vakuum eingeengt und der Rückstand im Vakuum fraktioniert destilliert.

Man erhält so 23,2 g (72,4 % der Theorie) 2-Trifluormethoxy-benzolsulfonyl-isocyaniddichlorid vom Siedepunkt Kp: 126 °C/1 mbar.

Analog Beispiel (IVa-1) können die folgenden Verbindungen der Formeln (IVa) bzw. (IV) erhalten werden:

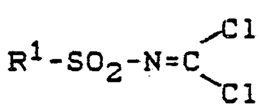 (IV)

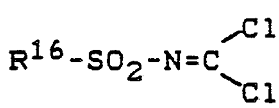 (IVa)

Tabelle 4

| Beisp.-Nr. | $R^{16}$ | Schmelzpunkt /[°C] |
|---|---|---|
| IVa-2 | 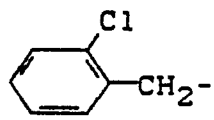 | 79 |
| IVa-3 | 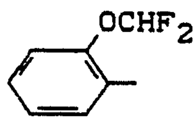 | |
| IVa-4 | 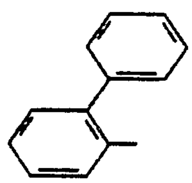 | |
| IVa-5 | 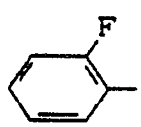 | |
| IVa-6 | 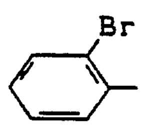 | 83 |
| IVa-7 | 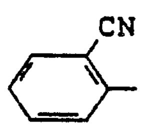 | |
| IVa-8 | 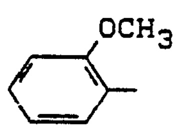 | |
| IVa-9 | 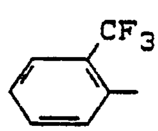 | |

Tabelle 4 - Fortsetzung

| Beisp.-Nr. | $R^{16}$ | Schmelzpunkt /[°C] |
|---|---|---|
| IVa-10 | 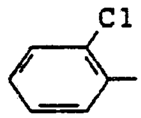 | 74 |
| IVa-11 | 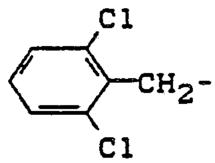 | 94 |
| IVa-12 | 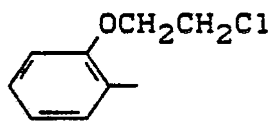 | |
| IV-13 | 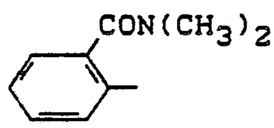 | |
| IV-14 | 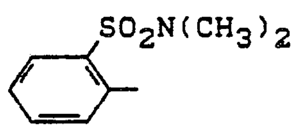 | |

Beispiel (IV-1)

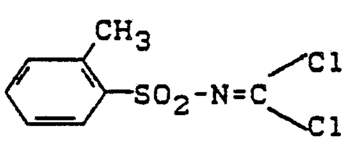

## Ansprüche

1) Verfahren zur Herstellung von Sulfonylisothioharnstoffen der allgemeinen Formel (I)

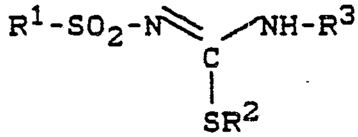 (I)

in welcher
$R^1$ für einen gegebenenfalls substituierten Rest aus der Reihe Aralkyl, Aryl und Heteroaryl steht,
$R^2$ für gegebenenfalls substituiertes Aryl steht, und
$R^3$ für einen gegebenenfalls substituierten aromatischen oder heteroaromatischen Rest steht,

dadurch gekennzeichnet, daß man Iminodithiokohlensäureester der Formel (II)

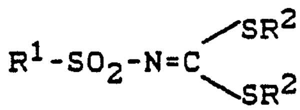

(II)

in welcher

$R^1$ und $R^2$ die oben angegebenen Bedeutungen haben,

mit Aminen der Formel (III)

$R^3$-$NH_2$ (III)

in welcher

$R^3$ die oben angegebenen Bedeutungen hat,

in Gegenwart von Säureakzeptoren und gegebenenfalls in Gegenwart von Verdünnungsmitteln bei Temperaturen zwischen 0 °C und 150 °C umsetzt.

2) Iminodithiokohlensäureester der allgemeinen Formel (IIa)

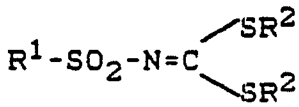

(IIa)

in welcher

$R^1$ für den Rest

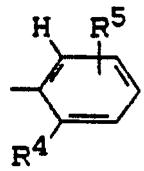

steht, worin

$R^4$ und $R^5$ gleich oder verschieden sind und für Wasserstoff, Halogen [wie insbesondere Fluor, Chlor, Brom und/oder Iod], Cyano, $C_1$-$C_6$-Alkyl, [welches gegebenenfalls durch Fluor, Chlor, Brom, Cyano, $C_1$-$C_4$-Alkoxy, $C_3$-$C_6$-Cycloalkyl oder Phenyl substituiert ist], für $C_1$-$C_4$-Alkoxy [welches gegebenenfalls durch Fluor, Chlor, Brom, Cyano oder $C_1$-$C_4$-Alkoxy substituiert ist], für Di-($C_1$-$C_4$-alkyl)-aminosulfonyl, für Phenyl oder Phenoxy, für $C_1$-$C_4$-Alkylcarbonylamino, $C_1$-$C_4$-Alkylamino-carbonyl-amino, Di-($C_1$-$C_4$-alkyl)-amino-carbonylamino, Di-($C_1$-$C_4$-alkyl)-aminocarbonyl oder für Di-($C_1$-$C_4$-alkyl)-aminosulfonylamino stehen, worin weiter

$R^1$ für den Rest

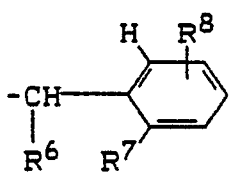

steht, worin

$R^6$ für Wasserstoff oder $C_1$-$C_4$-Alkyl steht,

$R^7$ und $R^8$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist] oder Di-($C_1$-$C_4$-alkyl)-aminosulfonyl stehen; worin weiter

$R^1$ für den Rest

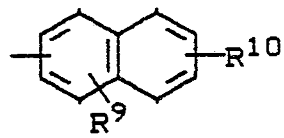

steht, worin
$R^9$ und $R^{10}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist] oder $C_1$-$C_4$-Alkoxy [welches gegebenenfalls durch Fluor und/oder Chlor substituiert ist], stehen; und
$R^2$ für einen Phenylrest steht, welcher gegebenenfalls substituiert ist durch einen oder mehrere Reste aus der Reihe
Halogen [wie insbesondere Fluor, Chlor, Brom und Iod], Cyano, Nitro, Hydroxy, Carboxy, $C_1$-$C_6$-Alkyl [welches gegebenenfalls durch Fluor, Chlor, Brom, Nitro, Cyano, Hydroxy, Carboxy, $C_1$-$C_4$-Alkoxy-carbonyl, $C_1$-$C_4$-Alkoxy, $C_1$-$C_4$-Alkylthio oder Phenyl substituiert ist], $C_3$-$C_6$-Cycloalkyl, $C_1$-$C_4$-Alkoxy [welches gegebenenfalls durch Fluor, Chlor, Brom, Cyano, Carboxy, $C_1$-$C_4$-Alkoxy, $C_1$-$C_4$-Alkylthio oder $C_1$-$C_4$-Alkoxy-carbonyl substituiert ist], $C_1$-$C_4$-Alkylthio [welches gegebenenfalls durch Fluor, Chlor, Brom, Cyano, Carboxy, $C_1$-$C_4$-Alkoxy-carbonyl substituiert ist], Amino, $C_1$-$C_4$-Alkyl-amino bzw. Di-($C_1$-$C_4$-alkyl)-amino [welches gegebenenfalls durch Fluor, Chlor, Brom, Cyano, Carboxy, $C_1$-$C_4$-Alkoxy oder $C_1$-$C_4$-Alkoxy-carbonyl substituiert sind], $C_1$-$C_4$-Alkyl-carbonyl-amino, $C_1$-$C_4$-Alkoxy-carbonylamino, (Di)-$C_1$-$C_4$-Alkylamino-carbonyl-amino, For myl, $C_1$-$C_4$-Alkyl-carbonyl, Benzoyl, $C_1$-$C_4$-Alkoxy-carbonyl, Phenoxy-carbonyl, Benzyloxycarbonyl, Phenyl [welches gegebenenfalls durch Fluor, Chlor, Brom, Cyano, Nitro, Hydroxy oder Methyl substituiert ist], Phenoxy, Phenylthio, Phenylsulfonyl, Phenylamino oder Phenylazo [welches gegebenenfalls durch Fluor, Chlor, Brom, Cyano, Nitro, Methyl und/oder Trifluormethyl substituiert sind], Pyridoxy oder Pyrimidoxy [welche gegebenenfalls durch Fluor, Chlor, Brom, Cyano, Nitro, Methyl und/oder Trifluormethyl substituiert sind], $C_1$-$C_4$-Alkyl-carbonyloxy, $C_1$-$C_4$-Alkoxy-carbonyloxy, $C_1$-$C_4$-Alkyl-amino-carbonyloxy und Di-($C_1$-$C_4$-alkyl)-amino-carbonyloxy, oder welcher gegebenenfalls durch eine Alkylenkette [welche gegebenenfalls verzweigt und/oder durch ein oder mehrere Sauerstoffatome unterbrochen ist] oder einen Benzorest [welcher gegebenenfalls durch Fluor, Chlor, Brom, Cyano, Nitro, Methyl und/oder Trifluormethyl substituiert ist] anelliert ist, wobei jedoch die folgenden Verbindungen ausgenommen sind:
N-(4-Methyl-benzolsulfonyl)-iminodithiokohlensäurediphenylester und N-(4-Chlor-benzolsulfonyl)-iminodithiokohlensäurediphenylester.

3) Verfahren zur Herstellung von Iminodithiokohlensäureestern der allgemeinen Formel (IIa), gemäß Anspruch 2, dadurch gekennzeichnet, daß man Iminokohlensäuredichloride der allgemeinen Formel (IV)

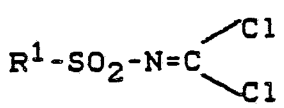 (IV)

in welcher
$R^1$ die oben unter Anspruch 2 angegebene Bedeutungen hat,
mit Verbindungen der Formel (V)
$R^2$-SM (V)
in welcher
$R^2$ die oben unter Anspruch 2 angegebenen Bedeutungen hat und
M für Wasserstoff oder ein Alkalimetallatom steht,
gegebenenfalls in Gegenwart von Säureakzeptoren gegebenenfalls in Gegenwart von Verdünnungsmitteln bei Temperaturen zwischen 10 °C und 100 °C umsetzt.